# EUROPEAN PATENT APPLICATION

(11) **EP 1 336 851 A1**
(43) Date of publication of application: **20.08.2003**
(21) Application number: 01994556.7
(22) Date of filing: 20.11.2001
(51) Int. Cl.: G01N 33/573

(54) **IMMUNOASSAY METHOD FOR MEMBRANE-BOUND MATRIX METALLOPROTEASE**

(30) Priority: 20.11.2000 JP 2000352491
(71) Applicant: Daiichi Fine Chemical Co., Ltd., Takaoka-shi, Toyama 933-8511 (JP)
(72) Inventor: AOKI, Takanori, DAIICHI FINE CHEMICAL CO., LTD., Takaoka-shi, Toyama 933-8511 (JP); YONEZAWA, Kayoko, DAIICHI FINE CHEMICAL CO., LTD, Takaoka-shi, Toyama 933-8511 (JP); FIJIMOTO, Noboru, DAIICHI FINE CHEMICAL CO., LTD, Takaoka-shi, Toyama 933-8511 (JP); OGAWA, Miwa, DAIICHI FINE CHEMICAL CO., LTD., Takaoka-shi, Toyama 933-8511 (JP); IWATA, Kazushi, DAIICHI FINE CHEMICAL CO., LTD., Takaoka-shi, Toyama 933-8511 (JP)
(74) Representative: Weber, Thomas, Dr.
(86) International application number: JP0110136
(87) International publication number: WO02041000

(57) **Abstract**

It is made possible to quantify MT1-MMP and to measure the enzymatic activity of MT1-MMP sensitively, accurately and quickly with simple operations and reagents. The MT1-MMP quantifications and the MT1-MMP enzymatic activity measurements can be carried out using quantitative immunoassays for MT1-MMP with anti-MT1-MMP Ab and reagents used therefor, further immunologically quantifying methods for a member selected from (i) MT1-MMP released and/or solubilized from cell membrane with a surfactant and/or a reducing agent and (ii) spontaneously solubilized MT1-MMP, reagents used therefor, solid-phased MT1-MMP, etc., thereby enabling the screening for a compound which promotes or inhibits the expression of MT1-MMP or a compound which promotes or inhibits the enzymatic activity of MT1-MMP, and facilitating the development and research for valuable pharmaceutical drugs. There are also provided a useful testing drug for cancer or cancer metastasis.

SEQUENCE LISTING

<110> Daiichi Fine Chemical Co., Ltd.

<120> Immunoassay for Membrane-type Matrix Metalloproteinases

<130> FC-98PCT

<140>
<141>

<150> JP 2000-352491
<151> 2000-11-20

<160> 7

<170> PatentIn Ver. 2.0

<210> 1
<211> 21
<212> DNA
<213> Artificial Sequence

<220>
<223> Description of Artificial Sequence:
      Oligonucleotide to act as a primer for PCR

<400> 1
gaagatctac gccatccagg g                                                21

<210> 2
<211> 25
<212> DNA
<213> Artificial Sequence

<220>
<223> Description of Artificial Sequence:
      Oligonucleotide to act as a primer for PCR

<400> 2
gaggtacctc agcccatcca gtccc                                            25

<210> 3
<211> 23
<212> DNA
<213> Artificial Sequence

<220>
<223> Description of Artificial Sequence:
      Oligonucleotide to act as a primer for PCR

<400> 3
taggtaccta cccgccgccc tcc                                              23

<210> 4
<211> 189
<212> PRT
<213> Homo sapiens

<400> 4
Cys Asp Gly Asn Phe Asp Thr Val Ala Met Leu Arg Gly Glu Met Phe
1               5                   10                  15

Val Phe Lys Glu Arg Trp Phe Trp Arg Val Arg Asn Asn Gln Val Met
            20                  25                  30

Asp Gly Tyr Pro Met Pro Ile Gly Gln Phe Trp Arg Gly Leu Pro Ala
        35                  40                  45

Ser Ile Asn Thr Ala Tyr Glu Arg Lys Asp Gly Lys Phe Val Phe Phe
    50                  55                  60

Lys Gly Asp Lys His Trp Val Phe Asp Glu Ala Ser Leu Glu Pro Gly
65                  70                  75                  80

Tyr Pro Lys His Ile Lys Glu Leu Gly Arg Gly Leu Pro Thr Asp Lys
                85                  90                  95

Ile Asp Ala Ala Leu Phe Trp Met Pro Asn Gly Lys Thr Tyr Phe Phe
            100                 105                 110

Arg Gly Asn Lys Tyr Tyr Arg Phe Asn Glu Glu Leu Arg Ala Val Asp
        115                 120                 125

Ser Glu Tyr Pro Lys Asn Ile Lys Val Trp Glu Gly Ile Pro Glu Ser
    130                 135                 140

Pro Arg Gly Ser Phe Met Gly Ser Asp Glu Val Phe Thr Tyr Phe Tyr
145                 150                 155                 160

Lys Gly Asn Lys Tyr Trp Lys Phe Asn Asn Gln Lys Leu Lys Val Glu
                165                 170                 175

Pro Gly Tyr Pro Lys Ser Ala Leu Arg Asp Trp Met Gly
            180                 185


<210> 5
<211> 189
<212> PRT
<213> Mus sp.

<400> 5

Cys Asp Gly Asn Phe Asp Thr Val Ala Met Leu Arg Gly Glu Met Phe
1               5                   10                  15

Val Phe Lys Glu Arg Trp Phe Trp Arg Val Arg Asn Asn Gln Val Met
            20                  25                  30

Asp Gly Tyr Pro Met Pro Ile Gly Gln Phe Trp Arg Gly Leu Pro Ala
        35                  40                  45

Ser Ile Asn Thr Ala Tyr Glu Arg Lys Asp Gly Lys Phe Val Phe Phe
    50                  55                  60

Lys Gly Asp Lys His Trp Val Phe Asp Glu Ala Ser Leu Glu Pro Gly
65                  70                  75                  80

Tyr Pro Lys His Ile Lys Glu Leu Gly Arg Gly Leu Pro Thr Asp Lys
                85                  90                  95

Ile Asp Ala Ala Leu Phe Trp Met Pro Asn Gly Lys Thr Tyr Phe Phe
            100                 105                 110

Arg Gly Asn Lys Tyr Tyr Arg Phe Asn Glu Glu Phe Arg Ala Val Asp
        115                 120                 125

Ser Glu Tyr Pro Lys Asn Ile Lys Val Trp Glu Gly Ile Pro Glu Ser
    130                 135                 140

Pro Arg Gly Ser Phe Met Gly Ser Asp Glu Val Phe Thr Tyr Phe Tyr
145                 150                 155                 160

Lys Gly Asn Lys Tyr Trp Lys Phe Asn Asn Gln Lys Leu Lys Val Glu
                165                 170                 175

Pro Gly Tyr Pro Lys Ser Ala Leu Arg Asp Trp Met Gly
            180                 185


<210> 6
<211> 189
<212> PRT
<213> Rattus sp.

<400> 6
Cys Asp Gly Asn Phe Asp Thr Val Ala Met Leu Arg Gly Glu Met Phe
1               5                   10                  15

Val Phe Lys Glu Arg Trp Phe Trp Arg Val Arg Asn Asn Gln Val Met
            20                  25                  30

Asp Gly Tyr Pro Met Pro Ile Gly Gln Phe Trp Arg Gly Leu Pro Ala
        35                  40                  45

Ser Ile Asn Thr Ala Tyr Glu Arg Lys Asp Gly Lys Phe Val Phe Phe
    50                  55                  60

Lys Gly Asp Lys His Trp Val Phe Asp Glu Ala Ser Leu Glu Pro Gly
65                  70                  75                  80

Tyr Pro Lys His Ile Lys Glu Leu Gly Arg Gly Leu Pro Thr Asp Lys
                85                  90                  95

Ile Asp Ala Ala Leu Phe Trp Met Pro Asn Gly Lys Thr Tyr Phe Phe
            100                 105                 110

Arg Gly Asn Lys Tyr Tyr Arg Phe Asn Glu Glu Phe Arg Ala Val Asp
        115                 120                 125

Ser Glu Tyr Pro Lys Asn Ile Lys Val Trp Glu Gly Ile Pro Glu Ser
    130                 135                 140

Pro Arg Gly Ser Phe Met Gly Ser Asp Glu Val Phe Thr Tyr Phe Tyr
145                 150                 155                 160

Lys Gly Asn Lys Tyr Trp Lys Phe Asn Asn Gln Lys Leu Lys Val Glu
                165                 170                 175

Pro Gly Tyr Pro Lys Ser Ala Leu Arg Asp Trp Met Gly
            180                 185

<210> 7
<211> 189
<212> PRT
<213> Oryctolagus cuniculus

<400> 7
Cys Asp Gly Asn Phe Asp Thr Val Ala Val Phe Arg Gly Glu Met Phe
1               5                   10                  15

Val Phe Lys Glu Arg Trp Phe Trp Arg Val Arg Asn Asn Gln Val Met
            20                  25                  30

Asp Gly Tyr Pro Met Pro Ile Gly Gln Leu Trp Arg Gly Leu Pro Ala
        35                  40                  45

Ser Ile Asn Thr Ala Tyr Glu Arg Lys Asp Gly Lys Phe Val Phe Phe
    50                  55                  60

Lys Gly Asp Lys His Trp Val Phe Asp Glu Ala Ser Leu Glu Pro Gly
65                  70                  75                  80

Tyr Pro Lys His Ile Lys Glu Leu Gly Arg Gly Leu Pro Thr Asp Lys
                85                  90                  95

Ile Asp Ala Ala Leu Phe Trp Met Pro Asn Gly Lys Thr Tyr Phe Phe
            100                 105                 110

Arg Gly Asn Lys Tyr Tyr Arg Phe Asn Glu Glu Leu Arg Ala Val Asp
        115                 120                 125

Ser Glu Tyr Pro Lys Asn Ile Lys Val Trp Glu Gly Ile Pro Glu Ser
    130                 135                 140

Pro Arg Gly Ser Phe Met Gly Ser Asp Glu Val Phe Thr Tyr Phe Tyr
145                 150                 155                 160

Lys Gly Asn Lys Tyr Trp Lys Phe Asn Asn Gln Lys Leu Lys Val Glu
                165                 170                 175

Pro Gly Tyr Pro Lys Ser Ala Leu Arg Asp Trp Met Gly
            180                 185

## Description

### Field of the Invention

The present invention relates to a quantitative immunoassay comprising the use of an antibody to membrane type matrix metalloproteinase (MT-MMP) (anti-MT-MMP antibody) and to an immunoassay reagent used therefor. The present invention further relates to a method for immunoiogical quantification of a member selected from the group consisting of (i) MT-MMP released and/or solubilized from cell membrane with a surfactant and/or a reducing agent and (ii) spontaneously solubilized MT-MMP and also to a reagent used therefor. More particularly, the present invention relates to a quantitative immunoassay for (method for immunologically quantifying) (a) MT-MMP released and/or solubilized from cell membrane with one or more members selected from the group consisting of surfactants and reducing agents or (b) spontaneously solubilized MT-MMP which comprises using an antibody to MT-MMP (such as anti-MT1-MMP Ab). The present invention further relates to a method for the detection of (A) a factor which modulate the expression of MT-MMP and (B) a factor for regulating the enzymatic activity of MT-MMP and to a pharmaceutical drug containing the factor. In another aspect, the present invention relates to a method for the release and the solubilization of MT-MMPs such as MT1-MMP from cell membrane with a surfactant and/or a reducing agent. The present invention furthermore relates to a method for measuring the enzymatic activity of MT-MMP and to a reagent used therefor.

### Background of the Invention

Matrix metalloproteinases (MMPs) are soluble enzymes having a structure where propeptide, enzymatically active site and hinge- and hemopexin-like site are fundamental domains. On the other hand, a membrane-type matrix metalloproteinases (MT-MMPs) forming a group of subfamily among the MMPs are membrane-type enzymes having membrane-associating domain at the C-terminal side and being localized in a form of being embedded on cell membrane. At present, genes of MMP-1, 2, 3, 7, 8, 9, 10, 11, 12, 13, 18, 19, 20, 21, 22, 23 and 26 and those of MMP-14, 15, 16, 17, 24 and 25 (MT1-, MT2-, MT-3, MT4-, MT-5 and MT6-MMP) are cloned and identified as solubilized MMP and membrane-type MMP, respectively.

### (Activation of proMMP-2 by MT1-MMP)

MMP-2 is an enzyme which digests gelatin, type IV collagen, etc. which are main components of basement membrane and it has been known that its activity is well correlated to invasion and metastasis of many human cancer tissues. ProMMPs (MMP-1, 3, 7, 8, 9, 10, 11 and 13) other than MMP-2 have cascade which is activated by endogenous serine protease or each of activated MMPs while proMMP-2 is characterized in having an activating mechanism different therefrom. Thus, proMMP-2 is bonded to MT1-MMP via TIMP-2 on cell membrane and adjacent another molecule of MT1-MMP cleaves a propeptide of proMMP-2 whereupon it is converted to an activated form.

### (MT1-MMP and invasion and metastasis of human cancer tissues)

MT1-MMP is well correlated to invasion and metastasis of human cancer tissues and deeply participated in activation mechanism of the expressed MMP-2 and, further, MT1-MMP itself also has an extracellular matrix degradation activity whereby it is likely to play an important role in invasion and metastasis of cancer. MT1-MMP has been reported to be widely expressed in many cancer cells such as breast cancer, colon cancer, gastric cancer, head and neck cancer, etc. and the correlation of the presence of MT1-MMP to degree of malignance of cancer, i.e. invasion and metastatic ability, has been receiving public attention.

### (MT1-MMP and chronic rheumatoid arthritis)

As an early-stage pathological picture of chronic rheumatoid arthritis (RA), angiogenesis and proliferation of synovial tissue are observed. Hyperplastic synovial surface layer cells produce MMP-1, 3, MT1-MMP and TIMP-1 while fibroblasts of lower layer produce MMP-2 and TIMP-2. Neutrophil invaded to synovial membrane and articular cavity produces MMP-8 and 9 and those MMPs and TIMPs are secreted into synoval fluid. Levels of MMP-1, 2, 3, 8 and 9 in RA synoval fluid are significantly higher than those in synoval fluid of osteoarthritis (OA) and are more than molecular numbers of TIMPs where MMPs have a dominant position. In addition thereto, destruction of articular cartilage of RA proceeds by MMP-1, 2, 3, 7, 8 and 13 expressed by the cartilage cells per se. Although the correlation between onset/progress of RA and MT1-MMP has been unknown, there is a possibility that destruction of articular cartilage by activation of proMMP-2 and ECM degradation activity of MT1-MMP per se participate in onset and progress of RA.

### (Molecular species of MT1-MMP)

MT-MMPs have a hydrophobic transmembrane domain at the side of C-terminal and are present in a form of being embedded on cell membrane via that and, therefore, they exhibit insolubility unlike other soluble MMPs. Actually, it has been reported that MT1-MMP is localized in cell membrane. It has been recently found that, in breast cancer strain treated with concanavalin A (Con A), MT1-MMP losing a hydrophobic transmembrane domain at the C-terminal side is present in a solubilized (shedding) form in its supernatant liquid of culture. It is a phenomenon that, during the process of apoptosis induced by concanavalin A, membrane-associated MT1-MMP is cleaved at the C-terminal side by any mechanism and is released from the membrane. Although the physiological function of the solubilized MT1-MMP has been unknown, various physiological functions are presumed as same as in the case of soluble MMPs. Thus, in the measurement of MT1-MMP, a method which is applicable to both membrane-associated MT1-MMP and soluble MT1-MMP seems to be appropriate.

In order to check how MT1-MMP is participated in invasion/metastasis of human cancer and pathology of chronic rheumatoid arthritis (RA), it is important to know the amount and the enzymatic activity of MT1-MMP contained in human body fluid such as blood, synoval fluid, abdominal dropsy, pleural effusion and urine or human tissues, culture cells, etc. For the measurement of MT1-MMP, there have been known a method where expression of mRNA is semi-quantitatively detected by a northern blotting, a method where it is semi-quantitatively detected by an RT-PCR, etc. but all of those methods are to reflect the expression level of gene and do not show the amount of functional MT1-MMP. Moreover, in those methods, samples to be tested are limited and, further, the methods are technically complicated for extraction of mRNA, etc. whereby confirmation of reproducibility is difficult.

For the detection of MT1-MMP, there is another method where evaluation is carried out using activation of proMMP2 as an indicator. The sample to be tested is analyzed by a gelatin zymography whereupon the activated MMP-2 is detected and the presence of MT1-MMP which is an activating factor therefor can be estimated. However, in this method, the presence of MT1-MMP is observed only indirectly and, in addition, a sample containing high concentration of protein such as blood component is unable to be directly subjected to a gelatin zymography.

It is also possible to detect MT1-MMP by Western blotting using anti-MT1-MMP antibody but, like in the case of zymography, it is not suitable for the detection of high protein concentration samples such as blood component and, moreover, its quantification and reproducibility are poor. As a method for the quantification of protein in living body, an immunological means has been known but the sample to be tested is limited to soluble substances only and it is not suitable for the quantitative determination of insoluble substance existing in cell membrane such as MT1-MMP.

An immunological method for quantitative determination of MT1-MMP has been reported by Harayama, et al. (Jpn. J. Cancer Res., 90:942-950 (1999)) but, in this method, although soluble MT1-MMP in the test sample can be measured, MT1-MMP bonded to the membrane cannot be measured. In addition, its measuring limit is high and, therefore, it is difficult to measure the soluble MT1-MMP which is present in a very small amount.

With regard to a method for the measurement of enzymatic activity of MT1-MMP, gelatin zymography has been known. Gelatin zymography is an excellent method for the detection of activity of MT1-MMP contained in non-purified sample to be tested since both fractionation of MT1-MMP and detection of activity can be carried out at the same time on electrophoresis. However, its operation is complicated and, in addition, the measured result is only within an extent of a semi-quantitative judgment.

As mentioned above, although detection or semi-quantitative determination of MT1-MMP is possible by northern blotting, RT-PCR, zymography and western blotting, all of them need complicated operation and give poor reproducibility whereby simple and convenient quantifying method and activity measuring method for MT1-MMP have not been available yet.

### Summary of the Invention

An object of the present invention is to provide methods for a quick quantitative determination of MT-MMP such as MT1-MMP with high sensitivity and accuracy, said method using a simple operation and reagent; methods for the enzymatic activity measurement of each MT-MMP including MT1-MMP and reagent kits therefor; and pharmaceuticals for suppressing the expression of MT-MMP such as MT1-MMP and for modulating MT-MMP enzymatic activity, said pharmaceutical containing an identified active substance, etc. by using such a method, reagent, etc.

The present invention is:
<1> to quantitate MT-MMP such as MT1-MMP in a sample to be tested, using an immunological method (immunological quantitating method for MT-MMP), particularly to quantitate a soluble MT-MMP in the sample to be tested;
<2> to provide a method for the preparation of a sample suitable for immunoassay by solubilizing the membrane-associated MT-MMP such as membrane-associated MT1-MMP in the sample to be tested;
<3> to quantitate the membrane-associated MT-MMP in a sample to be tested by an immunological method, said membrane-associated MT-MMP being converted to soluble MT-MMP by the above method <2>, or to quantitate the membrane-associated MT-MMP by an immunological method in a sample to be tested according to the above <1>, said membrane-associated MT-MMP being converted to soluble MT-MMP;
<4> to provide a method for the detection of factors, drugs, etc. which modulate the expressed amount of MT-MMP, said method using an immunoassay for MT-MMP and a pharmaceutical containing such a factor and drug;
<5> to specifically measure the enzymatic activity of immobilized MT-MMP wherein MT-MMP is selectively immobilized by an immunological method (assay for the enzymatic activity of MT-MMP); and
<6> to provide a method for the detection of factors, drugs, etc. which modulate the enzymatic activity of MT-MMP, said method using the assay for the enzymatic activity of MT-MMP according to the above <5> and a pharmaceutical containing such a factor and drug.

The present invention provides:
(1) A quantitative immunoassay for a MT-MMP member selected from the group consisting of MT-MMPs, comprising the use of an antibody to MT-MMP (anti-MT-MMP Ab);
(2) The quantitative immunoassay according to the aforementioned (1), wherein the assay comprises using an antibody member selected from the group consisting of antibodies to MMP-14 (MT1-MMP), antibodies to MMP-15 (MT2-MMP), antibodies to MMP-16 (MT3-MMP), antibodies to MMP-17 (MT4-MMP), antibodies to MMP-24 (MT5-MMP) and antibodies to MMP-25 (MT6-MMP);
(3) The quantitative immunoassay according to the aforementioned (1) or (2), wherein MT1-MMP is quantitatively immunoassayed with an antibody to MT1-MMP (anti-MT1-MMP Ab);
(4) The quantitative immunoassay according to any of the aforementioned (1) to (3), wherein the assay comprises using one or more members selected from the group consisting of surfactants and reducing agents;
(5) The quantitative immunoassay according to any of the aforementioned (1) to (4), wherein the antibody has at least one characteristic property selected from the group consisting of:
   (i) abilities of maintaining its immunoreactivity even in the presence of one or more members selected from the group consisting of surfactants and reducing agents and
   (ii) products from immunization with a denatured MT-MMP antigen having an epitope artificially exposed;
(6) The quantitative immunoassay according to the aforementioned (5), wherein the immunizing antigen is selected from the group consisting of MT1-MMP, MT2-MMP, MT3-MMP, MT4-MMP, MT5-MMP and MT6-MMP;
(7) The quantitative immunoassay according to the aforementioned (5), wherein the immunizing antigen is MT1-MMP;
(8) The quantitative immunoassay according to any of the aforementioned (1) to (7), wherein the antibody is monoclonal;
(9) The quantitative immunoassay according to any of the aforementioned (1) to (8), wherein the quantitatively assayable MT-MMP is at least one molecular species selected from the group consisting of:
   (i) molecules released and/or solubilized from cell membrane by treatments with one or more members selected from the group consisting of surfactants and reducing agents and
   (ii) molecules spontaneously released and/or solubilized from cell membrane;
(10) The quantitative immunoassay according to any of the aforementioned (1) to (9), wherein the assay comprises the following steps of:
   (a) releasing and/or solubilizing MT-MMP from cell membrane by treatment with one or more members selected from the group consisting of surfactants and reducing agents and
   (b) quantitatively immunoassaying for MT-MMP;
(11) The quantitative immunoassay according to any of the aforementioned (1), (2), (4) to (6) and (8) to (10), wherein the MT-MMP is a member selected from the group consisting of MT1-MMP, MT2-MMP, MT3-MMP, MT4-MMP, MT5-MMP and MT6-MMP;
(12) The quantitative immunoassay according to any of the aforementioned (1) to (11), wherein the MT-MMP is MT1-MMP;
(13) The quantitative immunoassay according to any of the aforementioned (4) to (12), wherein the surfactant is sodium dodecylsulfate (SDS);
(14) The quantitative immunoassay according to any of the aforementioned (4) to (12), wherein the reducing agent is 2-mercaptoethanol;
(15) An immunoassay reagent for MT-MMP which is used for the quantitative immunoassay as set forth in any of the aforementioned (1) to (14);
(16) The reagent according to the aforementioned (15), comprising an effective amount of an antibody to MT-MMP wherein the MT-MMP is selected from the group consisting of MT-MMPs;
(17) The reagent according to the aforementioned (15), wherein the MT-MMP is a member selected from the group consisting of MT1-MMP, MT2-MMP, MT3-MMP, MT4-MMP, MT5-MMP and MT6-MMP;
(18) The reagent according to the aforementioned (15), comprising an effective amount of an antibody member selected from the group consisting of antibodies to MT1-MMP, antibodies to MT2-MMP, antibodies to MT3-MMP, antibodies to MT4-MMP, antibodies to MT5-MMP and antibodies to MT6-MMP);
(19) The reagent according to the aforementioned (15), wherein the MT-MMP is MT1-MMP;
(20) The reagent according to the aforementioned (15), comprising an effective amount of anti-MT1-MMP Ab;
(21) The reagent according to any of the aforementioned (15) to (20), comprising an effective amount of one or more members selected from the group consisting of surfactants and reducing agents;
(22) A method for releasing and/or solubilizing MT-MMP, which comprises treating a cell membrane-containing sample with one or more members selected from the group consisting of surfactants and reducing agents to release and/or solubilize, from the cell membrane, a member selected from MT-MMPs;
(23) The method according to the aforementioned (22), wherein the surfactant is SDS;
(24) The method according to the aforementioned (22), wherein the reducing agent is 2-mercaptoethanol;
(25) The method according to any of the aforementioned (22) to (24), wherein the MT-MMP is a member selected from the group consisting of MT1-MMP, MT2-MMP, MT3-MMP, MT4-MMP, MT5-MMP and MT6-MMP;
(26) The method according to any of the aforementioned (22) to (24), wherein the MT-MMP is MT1-MMP;
(27) A releasing and/or solubilizing agent for MT-MMP, which is used for (a) the quantitative immunoassay as set forth in any of the aforementioned (1) to (14) or (b) the method as set forth in any of the aforementioned (22) to (26);
(28) The agent according to the aforementioned (27), comprising an effective amount of one or more members selected from the group consisting of surfactants and reducing agents;
(29) The agent according to the aforementioned (28), wherein the surfactant is SDS;
(30) The agent according to the aforementioned (28), wherein the reducing agent is 2-mercaptoethanol;
(31) The agent according to any of the aforementioned (27) to (30), wherein the MT-MMP is a member selected from the group consisting of MT1-MMP, MT2-MMP, MT3-MMP, MT4-MMP, MT5-MMP and MT6-MMP;
(32) The agent according to any of the aforementioned (27) to (30), wherein the MT-MMP is MT1-MMP;
(33) A screening method which comprises using one or more elements selected from the group consisting of:
   (a) the quantitative immunoassay as set forth in any of the aforementioned (1) to (14),
   (b) the reagent as set forth in any of the aforementioned (15) to (21),
   (c) the method as set forth in any of the aforementioned (22) to (26), and
   (d) the agent as set forth in any of the aforementioned (27) to (32),
   and screening for a compound that promotes or inhibits the expression of a member selected from the group consisting of MT-MMPs;
(34) The screening method according to the aforementioned (33), wherein the method comprises the following steps:
   (1) screening in the presence of a candidate compound to be tested,
   (2) screening in the absence of said candidate compound, and
   (3) comparing the screening results from the step (1) with those from the step (2);
(35) The screening method according to the aforementioned (33) or (34), which comprises screening for a compound that promotes or inhibits the expression of MT-MMP wherein said MT-MMP is selected from the group consisting of MT1-MMP, MT2-MMP, MT3-MMP, MT4-MMP, MT5-MMP and MT6-MMP;
(36) The screening method according to the aforementioned (33) or (34), which comprises screening for a compound which promotes or inhibits the expression of MT1-MMP;
(37) A screening kit for a compound which promotes or inhibits the expression of a member selected from the group consisting of MT-MMPs, said screening kit being used for the screening method as set forth in any of the aforementioned (33) to (36);
(38) The screening kit according to the aforementioned (37), wherein the kit contains an effective amount of one or more elements selected from the group consisting of
   (a) an antibody to MT-MMP, said MT-MMP being selected from the group consisting of MT-MMPs,
   (b) one or more members selected from the group consisting of surfactants and reducing agents, and
   (c) a member selected from the group consisting of MT-MMP attached to a solid phase by an immunological method;
(39) The kit according to the aforementioned (37) or (38), for the screening of a compound which promotes or inhibits the expression of MT-MMP, wherein the MT-MMP is selected from the group consisting of MT1-MMP, MT2-MMP, MT3-MMP, MT4-MMP, MT5-MMP and MT6-MMP;
(40) The kit according to the aforementioned (37) or (38), for the screening of a compound which promotes or inhibits the expression of MT1-MMP;
(41) A measuring method which comprises assaying for the enzymatic activity of MT-MMP wherein said MT-MMP is attached to a solid phase by an immunological method;
(42) The method according to the aforementioned (41), wherein the enzymatic activity of MT-MMP is quantitatively measured;
(43) The method according to the aforementioned (41) or (42), wherein the solid phase MT-MMP is formed by selectively binding to a solid phase via anti-MT-MMP Ab;
(44) The method according to any of the aforementioned (41) to (43), wherein the MT-MMP is a member selected from the group consisting of MT1-MMP, MT2-MMP, MT3-MMP, MT4-MMP, MT5-MMP and MT6-MMP;
(45) The method according to any of the aforementioned (41) to (43), wherein the MT-MMP is MT1-MMP;
(46) The method according to any of the aforementioned (41) to (45), wherein the MT-MMP is at least one molecular species selected from the group consisting of
   (i) molecules released and/or solubilized from cell membrane by treatments with one or more members selected from the group consisting of surfactants and reducing agents and
   (ii) molecules spontaneously released and/or solubilized from cell membrane;
(47) The method according to any of the aforementioned (41) to (46), wherein the method comprises the following steps:
   (a) releasing and/or solubilizing MT-MMP from cell membrane by treatments with one or more members selected from the group consisting of surfactants and reducing agents, and
   (b) assaying for the enzymatic activity of MT-MMP;
(48) Solid phase MT-MMP wherein MT-MMP is attached to a solid phase by an immunological method;
(49) The solid phase MT-MMP according to the aforementioned (48), wherein MT-MMP is selectively bound to a solid phase via anti-MT-MMP Ab;
(50) The solid phase MT-MMP according to the aforementioned (49), wherein the anti-MT-MMP Ab is an antibody which recognizes a hemopexin-like domain of MT-MMP;
(51) The solid phase MT-MMP according to any of the aforementioned (48) to (50), wherein MT-MMP is a member selected from the group consisting of MT1-MMP, MT2-MMP, MT3-MMP, MT3-MMP, MT4-MMP, MT5-MMP and MT6-MMP;
(52) The solid phase MT-MMP according to any of the aforementioned (48) to (50), wherein MT-MMP is MT1-MMP;
(53) A screening method for a compound which promotes or inhibits the enzymatic activity of MT-MMP which comprises using (i) the method as set forth in any of the aforementioned (41) to (47) or (ii) the solid phase MT-MMP as set forth in any of the aforementioned (48) to (52);
(54) The screening method according to the aforementioned (53), wherein the method comprises the following steps:
   (1) screening in the presence of a candidate compound to be tested,
   (2) screening in the absence of said candidate compound, and
   (3) comparing the screening results from the step (1) with those from the step (2);
(55) A screening kit for a compound which promotes or inhibits the enzymatic activity of MT-MMP, said kit being used for the method as set forth in any of the aforementioned (41) to (47);
(56) The kit according to the aforementioned (55), comprising an effective amount of solid phase MT-MMP as set forth in any of the aforementioned (48) to (52);
(57) A pharmaceutical drug or composition comprising an effective amount of one or more elements selected from the group consisting of
   (a) a compound which is obtained or identified by the method as set forth in any of the aforementioned (33) to (36), said compound which promotes or inhibits the expression of a member selected from the group consisting of MT-MMPs,
   (b) a compound which is obtained or identified by the use of the kit as set forth in any of the aforementioned (37) to (40), said compound which promotes or inhibits the expression of a member selected from the group consisting of MT-MMPs,
   (c) a compound which is obtained or identified by the method as set forth in the aforementioned (53) or (54), said compound which promotes or inhibits the enzymatic activity of MT-MMP, and
   (d) a compound which is obtained or identified by the use of the kit as set forth in the aforementioned (55) or (56), said compound which promotes or inhibits the enzymatic activity of MT-MMP;
(58) The pharmaceutical drug or composition according to the aforementioned (57), comprising an effective amount of
   (i) a compound that promotes or inhibits the expression of MT-MMP, said MT-MMP being selected from the group consisting of MT1-MMP, MT2-MMP, MT3-MMP, MT4-MMP, MT5-MMP and MT6-MMP, or
   (ii) a compound that promotes or inhibits the enzymatic activity of said MT-MMP;
(59) The pharmaceutical drug or composition according to the aforementioned (57), comprising an effective amount of
   (i) a compound that promotes or inhibits the expression of MT1-MMP, or (ii) a compound that promotes or inhibits the enzymatic activity of MT1-MMP;
(60) A diagnostic or testing drug for cancer or cancer metastasis, or for the progress degree of rheumatoid arthritis or Alzheimer's disease, enabling an assay for a member selected from the group consisting of MT-MMPs with (i) the quantitative immunoassay as set forth in any of the aforementioned (1) to (14) or (ii) the method as set forth in any of the aforementioned (41) to (47);
(61) The diagnostic or testing drug according to the aforementioned (60), with which
   (A) the level of MT-MMP is used as an indicator for evaluating cancer malignancy, for predicting cancer metastatic potential, or for assessing the progression level of rheumatoid arthritis or Alzheimer's disease, said MT-MMP being at least one molecular species selected from
      (i) molecules released and/or solubilized from cell membrane by treatments with one or more members selected from surfactants and reducing agents, and
      (ii) molecules spontaneously released and/or solubilized from cell membrane,
      or
   (B) the enzymatic activity level of MT-MMP is used as an indicator for evaluating cancer malignancy, for predicting cancer metastatic potential, or for assessing the progression level of rheumatoid arthritis or Alzheimer's disease, said MT-MMP being at least one molecular species selected from
      (iii) molecules released and/or solubilized from cell membrane by treatments with one or more members selected from surfactants and reducing agents, and
      (iv) molecules spontaneously released and/or solubilized from cell membrane;
(62) The diagnostic or testing drug according to the aforementioned (60) or (61), enabling an assay for MT-MMP selected from the group consisting of MT1-MMP, MT2-MMP, MT3-MMP, MT4-MMP, MT5-MMP and MT6-MMP;
(63) The diagnostic or testing drug according to the aforementioned (60) or (61), enabling an assay for MT1-MMP;
(64) The diagnostic or testing drug according to any of the aforementioned (60) to (62), comprising an effective amount of one or more antibodies selected from the group consisting of antibodies to MT1-MMP, antibodies to MT2-MMP, antibodies to MT3-MMP, antibodies to MT4-MMP, antibodies to MT5-MMP and antibodies to MT6-MMP;
(65) The diagnostic or testing drug according to any of the aforementioned (60) to (64), comprising an effective amount of an antibody to MT1-MMP;
(66) The diagnostic or testing drug according to any of the aforementioned (60) to (65), enabling an assay for the enzymatic activity of MT1-MMP; and
(67) Quantitative immunoassays, immunoassay reagents, screening methods, screening kits, enzymatic activity assays, MT-MMP immunologically attached to a solid phase, pharmaceutical drugs or compositions, diagnostic or testing drugs, and others, according to any of the aforementioned (1) to (66), wherein the antibody recognizes the hemopexin-like domain of MT-MMP.
   The present invention preferably provides quantitative immunoassays, immunoassay reagents, screening methods, screening kits, enzymatic activity assays, MT-MMP immunologically attached to a solid phase, pharmaceutical drugs or compositions, diagnostic or testing drugs, and others for soluble MT1-MMP.
   The present invention further provides:
(68) An antibody which is reactive to MT-MMP wherein the MT-MMP is selected from the group consisting of MT-MMPs, said antibody having at least one characteristic property selected from the group consisting of
   (i) abilities of maintaining its immunoreactivity even in the presence of one or more members selected from the group consisting of surfactants and reducing agents, and
   (ii) products from immunization with a denatured MT-MMP antigen having an epitope artificially exposed;
(69) The antibody according to the aforementioned (68), which is selected from the group consisting of antibodies to MT1-MMP, antibodies to MT2-MMP, antibodies to MT3-MMP, antibodies to MT4-MMP, antibodies to MT5-MMP and antibodies to MT6-MMP;
(70) The antibody according to the aforementioned (68) or (69), wherein the antibody is monoclonal;
(71) The antibody according to any of the aforementioned (68) to (70), which recognizes a hemopexin-like domain of MT-MMP;
(72) The antibody according to any of the aforementioned (68) to (71), wherein the antibody is attached to a solid phase; and
(73) The antibody according to any of the aforementioned (68) to (71), wherein the antibody is labeled.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein are described for illustrative purposes, the disclosure of which is hereby incorporated by reference.

### Brief Description of the Drawings

Fig. 1 schematically shows an epitope mapping profile where a monoclonal antibody to MT1-MMP according to the present invention is used and Δ MT1-MMP (58 kDa), a hemopexin domain (30 kDa, 34 kDa) and catalytic domain (21 kDa) fragments prepared in Example 1-③ are antigens.
Fig. 2 schematically shows a cross-reacting test profile by Western blotting where a monoclonal antibody of MT1-MMP according to the present invention is used and each of MT1-MMP-, MT2-MMP (mouse)- and MT3-MMP-expressing COS-7 cell lysates (prepared in Example 1-② ) is an antigen.
Fig. 3 is an embodiment of a standard curve obtained by MT1-MMP sandwich EIA.
Fig. 4 shows an MT1-MMP activity assay profile for various solid-phase monoclonal anti-MT1-MMP antibodies.
Fig. 5 shows a quantitative assay profile for MT1-MMP in extracts prepared from cancer tissues and adjacent normal tissues. The data are shown for each sample.
Fig. 6 shows a correlation between the amount of MT1-MMP in extracts prepared from cancer tissues and groups into which the said cancer tissues are classified depending on occurrence of metastasis to lymph node.
Fig. 7 shows a dilution curve profile obtained by quantitative assays for MT1-MMP in culture supernatants prepared from cultivated human, mouse, rat and rabbit cell lines.
Fig. 8 shows an immunoreactivity test profile for anti-MT1-MMP monoclonal antibody to MT1-MMP in the presence and the absence of a surfactant and a reducing agent. MT1-MMP was subjected to SDS-PAGE, transferred to a membrane and made to react with each antibody.

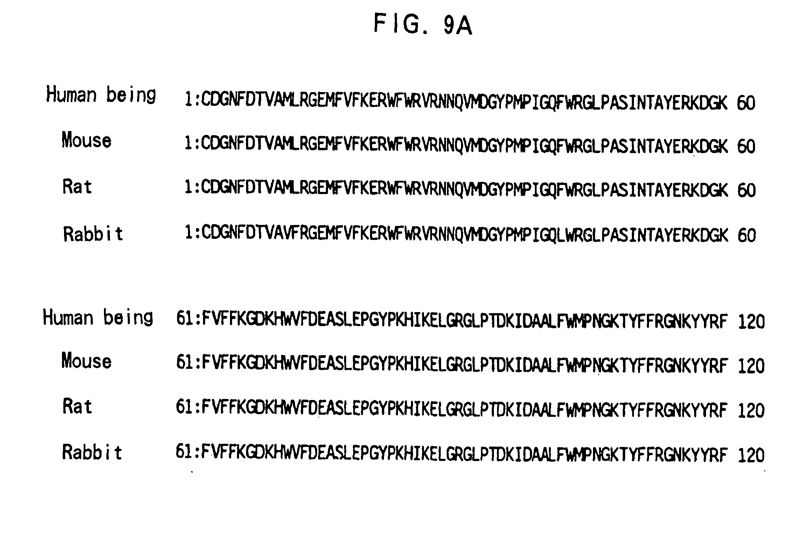

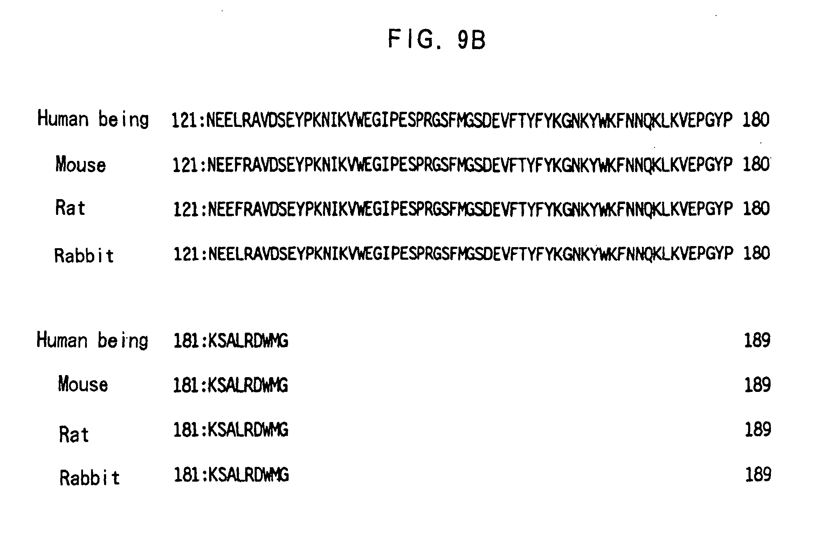

Fig. 9 shows amino acid sequence alignment comparisons among human, mouse, rat and rabbit-derived MT1-MMP hemopexin domains.

### Best Modes of Carrying out the Invention

The present invention provides an immunological quantifying method using an antibody to each of various MT-MMPs including an immunological quantitating method with an antibody to MT1-MMP (anti-MT1-MMP Ab). It particularly provides an immunological quantifying method for MT-MMP which comprises using a surfactant and/or a reducing agent. The present invention also provides an immunological quantifying method for the said MT-MMP which comprises the step of releasing and/or solubilizing MT-MMP from cell membrane with at least a member selected from the group consisting of a surfactant and a reducing agent. The present invention particularly provides an immunological quantifying method where MT-MMPS such as MT1-, MT2-, MT3-, MT4-, MT5- and MT6-MMP are assay targets. The antibody which specifically reacts with the said MT-MMP has at least a characteristic property selected from the group consisting of (i) abilities of maintaining the immunoreactivity in the presence of a surfactant and/or a reducing agent and (ii) products raised by using, as an immunizing antigen, artificially denatured MT-MMP such as artificially denatured MT1-MMP wherein one or more epitopes have been exposed. The preferable MT-MMP includes MT1-MMP. Among MT-MMPs such as MT1-, MT2-, MT3-, MT4-, MT5- and MT6-MMP, embodiments will be given for MT1-MMP in more detail, although it will be understood that other MT-MMPs such as MT2-, MT3-, MT4-, MT5- and MT6-MMP will be also able to be applied.

In an embodiment, the present invention provides an immunological quantifying method for MT1-MMP, characterized in that the immunologically, quantitatively assayable MT1-MMP is any of the following molecular species:
(i) molecules which are released and solubilized from cell membrane using a surfactant and/or a reducing agent; and
(ii) molecules which are spontaneously released and solubilized from cell membrane.

When the immunological quantitating method for the said MT1-MMP according to the present invention is used, there are provided a screening method and a screening kit for a compound which promotes or inhibits the expression of MT1-MMP. Thus, in accordance with the present invention, there is also provided a pharmaceutical which comprises the compound which promotes or inhibits the expression of MT1-MMP, said compound being identified or obtained by the said screening method or the screening kit. Further, in accordance with the immunological quantitating method for the said MT1-MMP according to the present invention, there are also provided a test drug for cancer or cancer metastasis and a testing drug for checking the progress level of rheumatoid arthritis or Alzheimer's disease. In an embodiment of the present invention, there are provided a method for the enzymatic activity measurement of MT1-MMP which comprises using MT1-MMP solid-phased by an immunological method and a reagent used therefor. Particularly, there are provided a method for measuring the enzymatic activity of MT1-MMP which comprises using MT1-MMP selectively solid-phased on a reactor via anti-MT1-MMP Ab and a reagent used therefor. Thus, in the present invention, there are also provided a screening method and a screening kit for a compound which promotes or inhibits the enzymatic activity of MT1-MMP using the said method for the measurement of MT1-MMP or the reagent used therefor. There is further provided a pharmaceutical which comprises an effective amount of the said compound which promotes or inhibits the enzymatic activity of MT1-MMP. Similarly, there are furthermore provided the above-mentioned methods, reagents, kits, etc. for other MT-MMPs in place of MT1-MMP.

As used herein, "membrane-type 1 matrix metalloproteinase" (or "MT1-MMP") is the enzyme also called matrix metalloproteinase-14 (MMP-14; MEROPS ID: M10.014), and one reported as the product of a gene occupying a chromosomal locus, 14 in humans (C. Mignon et al., Gemomics, 28: pp.360-361 (1995)), of which existence, detailed structure and profiles have been verified and demonstrated by successful DNA cloning and recombinant protein expression thereof (H. Sato et al., Nature, 370: pp.61-65 (1994); T. Takino et al., Gene, 155: pp.293-298 (1995); GenBank^{TM} accession number: D26512). The presence of MT1-MMP has been detected in the dogs, goats, rabbits, wild boars, mice, etc., in addition to humans. Human MT1-MMP cDNA encodes 582 amino acid residues (EMBL accession No. D26512, E09720 & E10297; SWISS-PROT: P50281), of which structure is composed of a signal peptide followed by a propeptide domain, an insert sequence composed of 10 specific amino acid residues similar to stromelysin-3 (a potential sequence of a furin-like enzyme recognition site), a core enzyme domain having a potential site with a zinc binding site, a hinge domain, a hemopexin-like domain encompassing a transmembrane domain and a hydrophobic C-terminal transmembrane domain. MT1-MMP may also be present as a species detached (released) from the membrane by cleavage at the C-terminal side via some mechanism, i.e., as solubilized MT1-MMP (soluble MT1-MMP form). Similarly, the corresponding species have been also known for other MT2-, MT3-, MT4-, MT5- and MT6-MMP, which is obvious for persons skilled in the art by appropriate reference to documents.

As used herein, the term "antibody" is used in the broadest sense and may cover a single species of monoclonal antibodies, antibody compositions having a specificity to various epitopes, further antibody fragments including Fv and others, and diabody molecules, particularly as long as they have desirable biological actions. Especially preferable antibodies include monoclonal antibodies. Monoclonal anti-MT1-MMP antibodies are suitably utilized in the present invention. It is apparent that other monoclonal anti-MT-MMP antibodies are utilizable herein.

As used herein, the term "monoclonal antibody" can be used in the broadest sense and may cover a single species of desirable monoclonal antibodies against MT1-MMP proteins or MT1-MMP protein fragments and monoclonal antibody compositions (or mixtures) having a specificity to various epitopes thereof, further monovalent or polyvalent antibodies, and also those which are intact molecules or fragments and derivatives thereof, including F(ab')₂, Fab' and Fab fragments, and also chimeric antibodies, hybrid antibodies each having at least two antigen or epitope binding sites, or bispecific recombinant antibodies (e.g., quadromes, triomes, etc.), interspecies hybrid antibodies, anti-idiotypic antibodies and those which have been chemically modified or processed and must be regarded as derivatives of these antibodies and further which may be produced either by adopting cell fusion or hybridoma techniques or antibody engineering or by using synthetical or semisynthetical techniques in known manner, which may be prepared either by the known conventional methods in view of antibody production or by recombinant DNA techniques, and which have neutralizing or binding properties with respect to the target antigen substances or target epitopes described and defined herein.

Monoclonal antibodies prepared against antigenic substances are produced by any method capable of providing production of antibody molecules by a series of cell lines in culture. The modifier "monoclonal" indicates the character of the antibody as being obtained from a substantially homogeneous population of antibodies, and is not to be construed as requiring production of the antibody by any particular method. The individual antibodies are those containing a population of identical antibodies except for possible naturally occurring mutations that may be present in minor amounts. Monoclonal antibodies are highly specific, being directed against a single antigenic site. In contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated or little contaminated by other immunoglobulins. The monoclonal antibodies included within the scope of the invention include hybrid and recombinant antibodies. They are obtainable by substituting a constant domain of an antibody for a variable domain (e.g., "humanized" antibodies), or a heavy chain for a light chain, by substituting a chain from one species with a chain from another species, or by fusing to heterogeneous proteins, regardless of species of origin or immunoglobulin class or subclass designation, so long as they exhibit the desired biological activity (e.g., U.S. Pat. No. 4,816,567; Monoclonal Antibody Production Techniques and Applications, pp.79 to 97, Marcel Dekker, Inc., New York, 1987; etc.).

Preferable techniques for producing monoclonal antibodies include, for example, the methods using hybridoma cells (G. Kohler and C. Milstein, Nature, 256, pp.495 to 497 (1975)); the methods using human B cell hybridomas (Kozbor et al., Immunology Today, 4, pp.72 to 79 (1983); Kozbor, J. Immunol., 133, pp.3001 (1984); Brodeur et al., Monoclonal Antibody Production Techniques and Applications, pp.51 to 63, Marcel Dekker, Inc., New York (1987); triome methods; EBV-hybridoma methods (Cole et al., Monoclonal Antibodies and Cancer Therapy, Alan R. Liss, Inc., pp.77 to 96 (1985)) (techniques for production of human monoclonal antibodies); U.S. Pat. No. 4,946,778 (techniques for production of single-chain antibodies), as well as the following documents: S. Biocca et al., EMBO J, 9, pp.101 to 108 (1990); R.E. Bird et al., Science, 242, pp.423 to 426 (1988); M.A. Boss et al., Nucl. Acids Res., 12, pp.3791 to 3806 (1984); J. Bukovsky et al., Hybridoma, 6, pp.219 to 228 (1987); M. DAINO et al., Anal. Biochem., 166, pp.223 to 229 (1987); J.S. Huston et al., Proc. Natl. Acad. Sci. USA, 85, pp.5879 to 5883 (1988); P.T. Jones et al., Nature, 321, pp.522 to 525 (1986); J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., Proc. Natl. Acad. Sci. USA, 81, pp.6851 to 6855 (1984); V.T. Oi et al., BioTechniques, 4, pp.214 to 221 (1986); L. Riechmann et al., Nature, 332, pp.323 to 327 (1988); A. Tramontano et al., Proc. Natl. Acad. Sci. USA, 83, pp.6736 to 6740 (1986); C. Wood et al., Nature, 314, pp.446 to 449 (1985); Nature, 314, pp.452 to 454 (1985), or documents quoted therein (the disclosures of which are incorporated herein by reference).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they have the desirable biological activity (U.S. Pat. No. 4,816,567; Morrison et al., Proc. Natl. Acad. Sci. USA, 81, pp.6851 to 6855 (1984)).

Described herein below is the production of antibodies, including embodiments of monoclonal antibodies. It goes without saying that the monoclonal antibody to be used in the present invention may be a product obtained by adoptions of cell fusion techniques (e.g., Kohler, G. & Milstein, C., Nature, 256: 495 (1975), etc.) with myeloma cells.

The monoclonal antibodies to be used in the present invention can be produced by the following processes:
1. Preparation of immunogenic antigens (immunogens)
2. Immunization of animals with immunogenic antigens
3. Preparation of myeloma cells
4. Cell fusion between antibody-producing cells and myeloma cells
5. Selection and cloning of hybridomas (hybrid cells)
6. Production of monoclonal antibodies

### 1. Preparation of immunogenic antigens

The antigen as used herein includes MT-MMPs, including, for example, soluble MT1-MMP protein fragments and derivative products thereof, as disclosed herein above. Examples of the antigen are suitable synthetic oligopeptides which are chemically synthesized, based on determined sequence information on the sequenced MT1-MMP protein. Alternatively, on the basis of the said information, appropriate gene libraries may be constructed, or a publicly known or readily available gene library may be appropriately used, and gene engineering operations including recombinant DNA techniques can be applied whereupon the MT1-MMP hemopexin domain, the MT1-MMP core enzyme domain having a potential site with a zinc binding site, the soluble protein of recombinant MT1-MMP, etc. will be obtainable and utilizable. For the antigen, MT1-MMP which is artificially denatured to expose its epitope may be preferably used. The above means may be similarly carried out for other MT-MMPs as well.

The gene recombination techniques (recombinant DNA techniques) can be carried out by the methods described in, for example, J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); The Japanese Biochemical Society (JBS) (Ed.), "Zoku-Seikagaku Jikken Koza 1, Idenshi Kenkyuhou II", Tokyo Kagaku Dojin Co. Ltd., Japan (1986); JBS (Ed.), "Shin-Seikagaku Jikken Koza 2, Kakusan III (Kumikae DNA Gijutsu)", Tokyo Kagaku Dojin Co. Ltd., Japan (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); J. H. Miller ed., "Methods in Enzymology", Vol. 204, Academic Press, New York (1991); R. Wu et al. ed., "Methods in Enzymology", Vol. 218, Academic Press, New York (1993); S. Weissman (ed.), "Methods in Enzymology", Vol. 303, Academic Press, New York (1999); J. C. Glorioso et al. (ed.), "Methods in Enzymology", Vol. 306, Academic Press, New York (1999), etc., or by methods described in the references quoted therein or methods substantially equivalent thereto or modified methods thereof (the disclosures of which are incorporated herein by reference).

Although the antigen may be used to immunize animals after being mixed with a suitable adjuvant without any modifications, it can be used after formation of immunogenic conjugates. For instance, the antigen for such an immunogen may be selected from fragmented molecules derived from MT-MMPs (such as MT1-MMP), synthetic polypeptide fragments which are prepared via selecting characteristic sequence areas based on the MT-MMP amino acid sequences followed by design and chemical synthesis. The fragments may be coupled with various carrier proteins via suitable coupling agents to form immunogenic conjugates such as hapten-proteins. The immunogenic conjugates can be used to design monoclonal antibodies that can react with (or recognize) specific sequences exclusively. A cysteine residue or others can be added to the polypeptide thus designed so as to prepare an immunogenic conjugate easily. To couple with a carrier protein or the like, the carrier protein is first activated. This activation may include incorporation of an activated binding group thereinto, etc. The activated binding groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, and bacterial components such as BCG.

### 2. Immunization of animals with immunogenic antigens

Animals can be immunized by methods known to those skilled in the art and according to techniques as described in, for example, Shigeru Muramatsu et al. ed., "Jikken-seibutsugaku-koza 14, Men-eki-seibutsu-gaku", Maruzen Co. Ltd., Japan, (1985); The Japanese Biochemical Society (Ed.), "Zoku-seikagakujikken-kouza 5, Men-eki-seikagaku-kenkyuho", Tokyo Kagaku Dojin Co. Ltd., Japan (1986); The Japanese Biochemical Society (Ed.), "Shin-seikagaku-jikken-kouza 12, Bunshi-men-eki-gaku III (Kougen-koutai-hotai)", Tokyo Kagaku Dojin Co. Ltd., Japan (1992); etc., the disclosures of which are hereby incorporated by reference. Immunization can be performed in a mammal, for example, by one or more injections of an immunizing agent (and, if desired, an adjuvant). Typically, the immunizing agent and/or adjuvant will be injected in the mammal by multiple subcutaneous or intraperitoneal injections. The immunizing agent may include the aforementioned antigen peptides or MMPs including, for example, MT1-MMP proteins and their fragments. It may be useful to conjugate the immunizing agent to a protein known to be immunogenic in the mammal being immunized. Examples of such immunogenic proteins which may be employed include the aforementioned carrier proteins. The adjuvant to be used with the antigen includes Freund's complete adjuvant, Ribi adjuvant, pertussis vaccine, BCG, lipid A, liposome, aluminium hydroxide, silica, etc.

The Immunization is carried out with suitable animals, including mice such as BALB/c, hamsters, and others. The antigen dose is, for example, about 1 to 400 µg/animal for mice. Generally, the antigen is injected intraperitoneally or subcutaneously into a host animal, followed by additional immunization by repeated courses wherein intraperitoneal, subcutaneous or intravenous administrations are carried out approximately 2 to 10 times at 1- to 4-week intervals, preferably 1- to 2-week intervals. For immunization, BALB/c mice, as well as F1 mice between BALB/c mice and other mice, etc. can be used. As required, the levels of animal immunization can be assessed by constructing an antibody titer measuring system and measuring the titer of an antibody. The antibody of the present invention may include those obtainable from such immunized animals, for example, anti-serum, polyclonal antibodies, etc.

### 3. Preparation of myeloma cells (plasmacytoma cell lines)

Immortal cell lines (tumor cell lines) to be used for cell fusion can be selected from non-immunoglobulin-producing cell lines. The cell lines to be used for cell fusion may include, for example, P3-NS-1-Ag4-1 (NS-1, Eur. J. Immunol., 6: 511-519, 1976), SP-2/0-Ag1 (SP-2, Nature, 276: 269 to 270,1978), mouse myeloma MOPC-21 cell line-derived P3-X63-Ag8-U1 (P3U1, Curr. topics Microbiol. Immunol., 81: 1 to 7, 1978), P3-X63-Ag8 (X63, Nature, 256: 495-497, 1975), P3-X63-Ag8-653 (653, J. Immunol., 123: 1548-1550, 1979), etc. 8-Azaguanine resistant mouse myeloma cell lines can be sub-cultured in a cell culture medium, such as Dulbecco's modified Eagle's medium (DMEM) and RPMI-1640, supplemented with antibiotics such as penicillin and amikacin, fatal calf serum (FCS) or others and 8-azaguanine (for example, 5 to 45 µg/ml). The specified number of cell lines can be prepared by passage the normal medium two to five days prior to cell fusion. The cell lines to be used may be cultured on the normal medium after the frozen and preserved cell lines have been completely thawed at about 37°C and have been washed on the normal medium such as RPMI-1640 three or more times, and the specified number of cell strains may be prepared.

### 4. Cell fusion between antibody-producing cells and myeloma cells

After animals such as mice are immunized according to the above step 2, their spleens are taken out in two to five days from final immunization, and the spleen cell suspension is obtained. In addition to the spleen cells, lymph node cells at various sites of organisms can be obtained and used for cell fusion. The spleen cell suspension thus obtained and the myeloma cell lines obtained by the above step 3 are placed in a medium such as minimum essential medium (MEM), DMEM and RPMI-1640 medium, followed by addition of a fusogen, such as polyethylene glycol (PEG). A widely-known fusogen can be used, including inactivated HVJ (Hemagglutinating virus of Japan, "Sendai virus") and the like. Preferably, 0.5 to 2 ml of 30 to 60% PEG can be added. PEG with molecular weights from 1,000 to 8,000 can be employed, more preferably, PEG with molecular weights from 1,000 to 4,000. The preferred concentration of PEG in the fusion medium is from 30 to 60%. As required, a small amount of dimethyl sulfoxide or the like is added to promote fusion. The ratio of spleen cells (lymphocytes) : myeloma cell lines to be used for fusion is preferably 1:1 to 20:1, and preferably falls within 4:1 to 7:1. The fusion reaction is conducted for 1 to 10 min, prior to the addition of a medium such as RPMI-1640 medium. Fusion reaction can be done several times. After fusion reaction, cells are separated by a centrifuge, then transferred to the selection medium.

### 5. Selection and cloning of hybridomas (hybrid cells)

The selection media include conventionally known "HAT medium", i.e., FCS-containing MEM, RPMI-1640 medium, etc., supplemented with hypoxanthine, aminopterin, and thymidine. The replacement method for the selection medium is to replenish an amount equivalent to the capacity dispensed to the medium plate on the following day, after which the medium is replaced by half an amount in HAT medium every one to three days. The replacement can be modified depending on situations. Eight to sixteen days after fusion, the medium may be replaced every one to four days with conventionally known "HT medium" wherein aminopterin is excluded. As a feeder cell, for example, mouse thymocyte can be used, which is sometimes effective.

The supernatant of the culture well with vigorously growing hybridoma is screened, for example, for assaying target antibodies, by using a predetermined peptide fragment as an antigen or by using a labeled anti-mouse antibody with a measuring system such as radioimmunoassay (RIA), enzyme-linked immunosorbent assay (ELISA), fluoroimmunoassay (FIA), luminescent immunoassay (LIA) and Western blotting, or by the fluorescence activated cell sorter (FACS), etc. The target antibody-producing hybridoma is cloned. Cloning is carried out by picking up colonies in the agar medium or by the limiting dilution. The limiting dilution is preferred. Cloning should be performed several times.

### 6. Production of monoclonal antibodies

The obtained hybridoma cells are cultured in a suitable growth medium such as FCS-containing MEM, RPMI-1640 medium or others, and a desired monoclonal antibody can be obtained from the culture supernatant. Large quantities of monoclonal antibodies can be produced by propagating hybridomas as ascites tumors, etc. In this case, each hybridoma is implanted intraperitoneally and propagated in a histocompatible animal isogenic to an animal from which the myeloma cell is derived. Or each hybridoma can be inoculated, for example, in nude mice, and propagated to produce the monoclonal antibody in the ascites of the animals. The produced monoclonal antibody can be collected from the ascetic fluid and obtained. Prior to implantation of hybridomas, the animal is pretreated intraperitoneally with mineral oils such as Pristane (2,6,10,14-tetramethylpentadecane). After the pretreatment, the hybridoma can be propagated therein and the ascitic fluid can be harvested. The ascitic fluid can be used as a monoclonal antibody without purification or after purification by conventionally known methods, including salting out such as precipitation with ammonium sulfate, gel filtration with Sephadex and the like, ion exchange chromatography, electrophoresis, dialysis, ultrafiltration, affinity chromatography, high-performance liquid chromatography, etc. The isolated or purified products can be employed as monoclonal antibodies. Preferably, the monoclonal antibody-containing ascitic fluid is fractionated with ammonium sulfate, separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize, etc.); affinity chromatography with immobilized protein A; hydroxyapatite chromatography; etc.

In addition, it is possible to use transgenic mice and other organisms including other mammals for expressing antibodies such as humanized antibodies against the immunogenic polypeptide products of the present invention.

It is also possible to produce antibodies with recombinant DNA techniques wherein the antibody thus obtained in a large amount is sequenced and/or a nucleotide sequence is employed which codes for the antibody obtained from the hybridoma cell line.

DNA coding for the monoclonal antibody is readily isolated and sequenced using conventional procedures (e.g., by using oligonucleotide probes that are capable of binding specifically to genes encoding the heavy or light chain of murine antibodies). Once isolated, the DNA may be placed, according to the aforementioned techniques, into expression vectors, which are then transfected into host cells such as CHO cells or COS cells. The DNA may be modified, for example, by substituting the coding sequence for human heavy- and light-chain constant domains for the homologous murine sequences (Morrison et al., Proc. Natl. Acad. Sci. USA, 81: 6581, 1984). Thus, chimeric and hybrid antibodies having a desired binding specificity can be prepared. Further, antibodies can be modified, including preparations of chimeric or hybrid antibodies by adaptations of known methods in synthetic protein chemistry, including those involving coupling agents as listed hereinbelow.

Humanized antibodies are achievable by known techniques in the art (e.g., Jones et al., Nature, 321: pp.522-525 (1986); Riechmann et al., Nature, 332: pp.323-327 (1988); Verhoeyen et al., Science, 239: pp.1534-1536 (1988)).

Human monoclonal antibodies can be achieved according to known techniques in the art. For producing human monoclonal antibodies, human myeloma cells and human-mouse hetero-myeloma cells are known in the art (Kozbor, J. Immunol., 133, pp.3001 (1984); Brodeur et al., Monoclonal Antibody Production Techniques and Applications, pp.51-63, Marcel Dekker, Inc., New York (1987)). Methods for making bispecific antibodies are known in the art (Millstein et al., Nature, 305: pp.537-539 (1983); WO93/08829 Traunecker et al., EMBO J., 10: pp.3655-3659 (1991); Suresh et al., "Methods in Enzymology", Vol. 121, pp.210 (1986)).

These antibodies may be treated with enzymes such as trypsin, papain, pepsin and others and occasionally be subjected to reduction to produce antibody fragments including Fab, Fab', and F(ab')₂. These antibody fragments may be occasionally used.

The antibodies may be employed in any known assay method, such as competitive binding assays, direct and indirect sandwich assays, and immunoprecipitation assays (Zola, Monoclonal Antibodies: A Manual of Techniques, pp.147-158, CRC Press, Inc., 1987).

Any method known in the art may be employed for separately conjugating the antibody to a detectable moiety. Such methods include those methods described in David et al., Biochemistry, Vol. 13, pp.1014-1021 (1974); Pain et al, J. Immunol. Meth., 40: pp.219-231 (1981); and "Methods in Enzymology", Vol. 184, pp.138-163 (1990). The antibody to be labeled with a marker may include IgG fractions, and specific binding fragments Fab' obtainable by reduction after pepsin digestion. The labels include enzymes (e.g., peroxidase, alkaline phosphatase, beta-D-galactosidase, etc.), chemical substances, fluorescences, radioisotopes, and the like, as disclosed herein below.

In the present invention, detection (including prediction) and measurement (assay) can be carried out by immunostaining including, for example, staining of tissues and cells, immunoassays including, for example, competitive and non-competitive immunoassays, FIA, LIA, RIA, ELISA, etc. The detection and measurement (assay) can also be carried with or without B-F separation. The detection and measurement is carried out preferably by radioimmunoassay and enzyme immunoassay, as well as sandwich assay. In the sandwich-type assay for example, one of the antibodies is set against MT-MMP hemopexin-like domains (such as MT1-MMP hemopexin-like domains), and the other against one of MT-MMPs (such as ordinary MT1-MMP) wherein one of both the antibodies is detectably labeled and the other antibody capable of recognizing the same antigen is immobilized on a solid phase. As required, incubation is carried out to sequentially react a sample to be assayed, labeled antibodies, and immobilized antibodies. After the non-binding antibodies are separated, the label is detected or measured. The amount of the measured label is proportional to the amount of an antigen, i.e., the amount of a soluble MT-MMP antigen (the amount of a soluble MT1-MMP antigen). This assay is referred to as simultaneous sandwich assay, forward sandwich assay, or reverse-sandwich assay, based on the difference according to the addition sequence of the insolubilized antibody and the labeled antibody. For example, washing, stirring, shaking, filtration, pre-extraction for antigen, and other treatments are optionally adopted in the measurement or assay process under specific conditions. The other assay conditions including the concentrations of specific reagents, buffers and others, temperatures, incubation times and the like can vary according to elements, such as the concentration of antigens in the sample, and the nature of samples to be measured. Any person ordinary skilled in the art can suitably select and determine optimal conditions effective for each assay while using the general experimentation and perform the selected measurement.

Various carriers on which antigens or antibodies can be immobilized are available in the art, and they can be arbitrarily and suitably selected in the present invention. For the immobilization, various carriers are known which can be used for antigen-antibody interactions. It goes without saying that any well-known carrier can be selected and used in the present invention. Preferred examples are inorganic materials including, for example, glass such as aminoalkylsilyl glass and other activated glass, porous glass, silica gel, silica-alumina, alumina, magnetized iron, magnetized alloy, etc.; organic polymer substances, such as polyethylene, polypropylene, polyvinyl chloride, polyvinylidene fluoride, polyvinyl acetate, polymethacrylate, polystyrene, styrene-butadiene copolymer, polyacrylamide, crosslinked polyacrylamide, styrene-methacrylate copolymer, polyglycidyl methacrylate, and acrolein-ethylene glycol dimethacrylate copolymer; cross-linked albumin, collagen, gelatin, dextran, agarose, crosslinked agarose, natural and modified cellulose (for example, cellulose, microcrystalline cellulose, carboxymethylcellulose, cellulose acetate, etc.), crosslinked dextran, polyamides (for example, nylon, etc.), polyurethane, polyepoxy resin, etc.; products obtained by emulsion polymerization of such organic polymer substances; cells, erythrocytes, etc.; and such substances having a functional group introduced thereinto, as required, by using a silane coupling agent, etc..

Also included are solid materials (bodies) such as filter paper, beads, inner walls of test containers such as test tubes, titer plates, titer wells, glass cells, cells made of synthetic materials such as plastic resin cells, glass rods, rods made of synthetic materials, rods thickened or thinned at the end, rods provided with a round protrusion or a flat protrusion at the end, thin-plated rods, and surfaces thereof.

Antibodies can be coupled with these carriers. Preferably antibodies (particularly monoclonal antibodies) which are obtainable according to the present invention and specifically reactive with antigens, may be coupled to such a carrier as mentioned above. Coupling between the carrier and those partners associated with these antigen-antibody interactions can be carried out by techniques including physical method such as adsorption; a chemical method using a coupling agent, etc. or an activated reactant; a method using a chemically interactional coupling.

The label may include enzymes, enzyme substrates, enzyme inhibitors, prosthetic groups, coenzymes, enzyme precursors, apoenzymes, fluorescent substances, pigments, chemoluminescent compounds, luminescent substances, coloring substances, magnetic substances, metal particles such as gold colloids, radioactive substances and the like. The enzyme may include dehydrogenases, oxidoreductases such as reductases and oxidases; transferases that catalyze the transfer of functional groups such as amino, carboxyl, methyl, acyl, and phosphate groups; hydrolases that hydrolyze bonds such as ester, glycoside, ether, and peptide bonds; lyases; isomerases; ligases; and the like. Plural enzymes may be used in a conjugated form for detection (for example, enzymatic cycling may also be utilizable). Typical radioactive isotopes for the label include [³²P]_{,} [¹²⁵I], [¹³¹I], [³H] , [¹⁴C], [³⁵S], etc. Typical enzymes for the label include peroxidases such as horseradish peroxidase; galactosidases such as E. coli beta-D-galactosidase; maleate dehydrogenases; glucose-6-phosphate dehydrogenases; glucose oxidases; gluocoamylases; acetylcholine esterases; catalases; alkaline phosphatases such as calf intestinal alkaline phosphatase and E. coli alkaline phosphatase, and the like. In the case where alkaline phosphatase is used, measurements can be done by monitoring or inspecting fluorescence, luminescence, etc., generated with substrates such as umbelliferone derivatives including 4-methylumbellipheryl phosphate, phenol phosphate derivatives including nitrophenyl phosphate, luciferin derivatives and dioxetane derivatives; enzymatic cycling systems utilizing NADP; etc. It is also possible to use a luciferin/luciferase system. When catalase is used, the reaction takes place with hydrogen peroxide to produce oxygen which can be detected with an electrode or the like. The electrode may be a glass electrode, an ionic electrode using an insoluble salt membrane, a liquid-membrane type electrode, a polymer membrane electrode and the like. The enzyme label may be replaced with a biotin label and an enzyme-labeled avidin (streptoavidin). For the label, a plurality of various kinds of labels or markers can be used. In this case, it is possible to perform plural measurements continuously or discontinuously and/or simultaneously or separately.

According to the present invention, signal formation may be done using enzyme-reagent combinations, such as combinations of horseradish peroxidase or other peroxidases with a member selected from 4-hydroxyphenylacetic acid, 1,2-phenylenediamine, tetramethylbenzidine, etc.; combinations of beta-D-galactosidases or glucose-6-phosphate dehydrogenases with a member selected from umbelliferyl galactosides, nitrophenyl galactosides, etc.; etc. The signal may be formed with those capable of enzymatically forming quinole compounds such as hydroquinone, hydroxybenzoquinone, and hydroxyanthraquinone; thiol compounds such as lipoic acid and glutathione; phenol derivatives; ferrocene derivatives; etc.

The fluorescent substances and chemiluminescent compounds may include fluorescein isothiocyanate; Rhodamine derivatives such as Rhodamine B isothiocyanate and tetramethyl Rhodamine isothiocyanate, dancyl chloride (5-(dimethylamino)-1-naphtalenesulfonyl chloride), dancyl fluoride, fluorescamine (4-phenylspiro[furan-2(3H),1'-(3'H)-isobenzofuran]-3,3'-dione), phycobiliprotein, acridinium salts; luminol compounds such as lumiferin, luciferase and aequorin; imidazoles, oxalic acid esters, rare earth chelate compounds, cumarin derivatives, etc.

The labelling can be accomplished by the reaction of a thiol group with a maleimide group, the reaction of a pyridyldisulfide group with a thiol group, the reaction of an amino group with an aldehyde group, etc. Additionally, it can be suitably selected from widely known methods, techniques which can be easily put into practice by an artisan skilled in the art, and any of modifications derived therefrom. The coupling agents used for producing the foregoing immunoconjugate or for coupling with carriers are also applicable and usable. The coupling agents include, for example, formaldehyde, glutaraldehyde, hexamethylene diisocyanate, hexamethylene diisothiocyanate, N,N'-polymethylene bisiodoacetamide, N,N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio)propionate (SPDP), N-succinimidyl 4-(N-maleimidometyl)cyclohexane-1-carboxylate (SMCC), N-sulfosuccinimidyl 4-(N-maleimidomethyl)-cyclohexane-1-carboxylate, N-succinimidyl (4-iodoacetyl)-aminobenzoate, N-succinimidyl 4-(1-maleimidophenyl)butyrate, N-(epsilon-maleimidocaproyloxy)succinimide (EMCS), iminothiolane, S-acetylmercaptosuccinic anhydride, methyl-3-(4'-dithiopyridyl)propionimidate, methyl-4-mercapto-butyrylimidate, methyl-3-mercaptopropionimidate, N-succinimidyl-S-acetylmercaptoacetate, etc.

According to the assay of the present invention, substances to be measured can be made to react sequentially with labeled antibody reagents (such as labeled monoclonal antibodies, including enzyme-labeled monoclonal antibodies, etc.) and then with antibodies coupled on a carrier, or all the members can be reacted each other simultaneously. The order of adding reagents (members) may vary depending on the type of carrier system selected. In the case where sensitized beads such as sensitized plastic beads are used, the labeled antibody regents (such as labeled antibodies including enzyme-labeled monoclonal antibodies) are first put into a suitable test tube, together with a sample containing substances to be measured, followed by addition of the sensitized beads (such as sensitized plastic beads). Measurement can be then carried out.

For quantitative measurements (and/or quantifying methods) according to the present invention, the immunological measurement (immunoassay) is applied. For the measurement (assay), the solid phase carriers used may include various materials and shapes which can be selected from balls, microplates, sticks, microparticles, test tubes, and others, made of polystyrene, polycarbonate, polypropylene, polyvinyl and other materials capable of adsorbing proteins such as antibodies.

The assay can be carried out in a suitable buffer system so as to maintain an optimal pH value (for example, between pH about 4 and about 9). The particularly preferred buffers may include acetate buffers, citrate buffers, phosphate buffers, Tris buffers, triethanolamine buffers, borate buffers, glycine buffers, carbonate buffers, Tris-HCl buffers, etc. The buffers can be used optionally in a mixed form at any ratio. Preferably, the antigen-antibody interaction is carried out at a temperature between about 0 and 60°C.

The labeled antibody reagents (e.g., monoclonal antibodies labeled with enzymes or others, etc.), the immobilized antibody reagents (coupled to a carrier), and substances to be assayed can be incubated until equilibrium is reached. However, the reaction may be stopped after limited incubation wherein the solid phase is separated from the liquid phase at a time point well before the antigen-antibody interaction equilibrates, and the level of labels (such as enzymes) existing in either of the liquid and solid phases may be measured. Measurement operation can be performed with automated measuring instruments. A luminescence detector, a photo detector or the like may be used to measure or detect indication signals generated as a result of substrate conversion by the action of an enzyme.

In the antigen-antibody interaction, adequate means can be taken so as to stabilize reagents to be used, samples to be assayed, and labels such as enzymes, respectively, and/or to stabilize antigen-antibody interactions per se. Further, proteins, stabilizers, surfactants, chelating agents or others can be added to incubation solutions for eliminating non-specific reaction, reducing inhibitory influences acting thereon, and/or activating assay reaction. The blocking techniques for preventing non-specific binding reaction, which techniques are generally employed in the art or well-known among the persons skilled in the art, may be employed. The blocking can be achieved by treatments with mammal normal serum, serum proteins, albumin, skim milk, fermented milk products, collagen, gelatin, or others. These methods or techniques can be used without any limitation so long as the use is for the purpose of preventing non-specific binding reaction.

Particularly in the enzyme immunoassays using anti-MT1-MMP Ab according to the present invention, enzyme immunoassay techniques are applicable without any limitation, which enzyme immunoassay techniques have been widely known in the art. The said enzyme immunoassays may include embodiments as disclosed in Eiji Ishikawa (editorially translated by), P. Tijssen, "Seikagaku Jikken Hou, Enzyme Immunoassays", Tokyo Kagaku Dojin Co. Ltd., Japan (1989) (original English language edition; P. Tijssen, "Practice and Theory of Enzyme Immunoassays" in R.H. Burdon and P.H. van Knippenberg (Ed.), "Laboratory Techniques in Biochemistry and Molecular Biology", Elsevier Science Publishers, Amsterdam, 1985), etc. They include, for example, the first antibody solid phase method, the double antibody technique, EMIT (enzyme multiplied immunoassay technique), enzyme channeling immunoassays, enzyme activity-modifier immunoassays, liposome membrane-enzyme immunoassays, sandwich methods, immunoenzyme metric assays and enzyme activity amplification immunoassays, etc., which may be either competitive or non-competitive.

The samples to be assayed according to the present invention may include various forms of solutions such as colloid solutions, non-fluid samples and the like. Preferably, the samples are biological samples including, for example, all organs and tissues, such as thymus, testis, intestine, kidney, brain, breast tissues, ovary, uterus, lungs, liver, stomach, pancreas, and esophagus; malignant tumors of such organs and tissues, including breast cancer, ovarian cancer, lung cancer, uterus cancer, colonic or rectal cancer, various sarcomas, etc.; blood, sera, plasma, synoval fluid, cerebrospinal fluid, saliva, amniotic fluid, urine, and other body fluids, cell culture medium, tissue culture medium, tissue homogenate, biopsy samples, tissues, cells, etc.

When an immunological quantification of MT-MMP (such as MT1-MMP) is carried out in accordance with the present invention, it has been found that MT-MMPs (such as MT1-MMP) can be measured as molecular species (soluble MT-MMP) released and/or solubilized from cell membrane. When the sample is appropriately treated with, for example, a member selected from a surfactant and a reducing agent, there is obtained a substance released and/or solubilized from cell membrane by some reason and the measurement of MT-MMP such as MT1-MMP can be effectively carried out. Thus, there is provided a technique where MT1-MMP and other MT-MMPs are released and/or solubilized from cell membrane with a member selected from a surfactant and a reducing agent. There is further provided a technique where MT-MMPs such as MT1-, MT2-, MT3-, MT4-, MT5- and MT6-MMP are released and/or solubilized from the cell membrane with a member selected from a surfactant and a reducing agent. When the said releasing and/or solubilizing technique is utilized, it is possible to subject MT-MMP, particularly released and/or solubilized MT-MMP, to an immunological quantification using an antibody to MT-MMP, particularly a monoclonal antibody (mAb) (such as anti-MT1-MMP Ab, anti-MT2-MMP Ab, anti-MT3-MMP Ab, anti-MT4-MMP Ab, anti-MT5-MMP Ab, and anti-MT6-MMP Ab).

The said surfactant includes any so far as it has an ability of releasing and/or solubilizing the MT-MMP from cell membrane. Preferably, it includes those which have no adverse action on the immunological quantitation of MT-MMP. Examples of the said surfactant are anionic surfactants and typical ones are alkaline metals salts of higher alkyl sulfuric acids. An example of the preferred surfactant is sodium dodecylsulfate (SDS). The said reducing agent may include agents having an ability of releasing and/or solubilizing the MT-MMP from cell membrane and agents acting on a sensitive group to oxidation-reduction (such as an S-S- bond) in the MT-MMP molecule. Preferably, it include those having no adverse action on the immunological quantitation of MT-MMP. The said reducing agents may be sulfur-containing organic compounds such as thioalcohols (e.g., 2-mercaptoethanol, dithiothreitol, etc.) and glutathione.

Preferably, both the surfactant and the reducing agent are used whereby MT-MMP can be released and/or solubilized from the membrane and MT-MMP, particularly released and/or solubilized MT-MMP, can be immunologically assayed (including quantitation) with anti-MT-MMP Ab or others. As a result, it is now possible to discriminate the released and/or solubilized MT-MMP from membrane-associated MT-MMP. Even in the case where detection or measurement is difficult due to bonding to the membrane, it is now possible to detect or measure the MT-MMP. Particularly, SDS is used as a surfactant and 2-mercaptoethanol is used as a reducing agent whereupon MT1-MMP can be released and/or solubilized. Further, anti-MT1-MMP Ab, particularly anti-MT1-MMP mAb, is used whereupon the said released and/or solubilized MT1-MMP can be immunologically quantitated.

In applying various analytic and quantitative assays including those individual immunological assays (immunoassays) to the measurements (assays) of the present invention, it is unnecessary to set up therefor any special condition, operation, etc. Assay systems for the targets of the present invention or target substances having a substantially equivalent activity thereto may be constructed by adaptations of technical consideration ordinarily given by artisans in the art over general conditions and operations suitable for each of the methods.

For details of those conventional technical methods, a variety of reviews, texts, books, etc. may be referred to. They are, for example, Hiroshi Irie (ed.), "Radioimmunoassay", Kodansha Ltd., Japan, 1974; Hiroshi Irie (ed.), "Zoku-Radioimmunoassay" (Radioimmunoassay; Second Edition), Kodansha Ltd., Japan, 1979; Eiji Ishikawa et al. (ed.), "Koso Meneki Sokuteiho" (Enzyme Immunoassays), Igaku-Shoin Ltd., Japan, 1978; Eiji Ishikawa et al. (ed.), "Koso Meneki Sokuteiho" (Enzyme Immunoassays) (2nd Edition), Igaku-Shoin Ltd., Japan, 1982; Eiji Ishikawa et al. (ed.), "Koso Meneki Sokuteiho" (Enzyme Immunoassays) (3rd Edition), Igaku-Shoin Ltd., Japan, 1987; Eiji Ishikawa, "Chou-koukanndo Koso Meneki Sokuteiho" (Supersensitive Enzyme Immunoassays), Japan Scientific Societies Press (JSSP), Japan, December 1993; H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991); etc. and references quoted in the above documents, the disclosures of which are incorporated herein by reference.

In the present invention, there is provided a screening method which comprises utilizing an immunological quantitating method of MT-MMP such as MT1-MMP to screen for a compound which promotes or inhibits the expression of MT-MMP such as the expression of MT1-MMP. In the said screening method, cultured MT-MMP (such as MT1-MMP)-expressing cells, transformed cells which express MT-MMP (such as MT1-MMP), transgenic animals such as transgenic mice, etc. are maintained in the presence of any physiologically active factor, drug, compound, etc. and compared with untreated cases. Amounts of the MT-MMP (such as MT1-MMP) in the sample are measured by the immunological quantitating method for the said MT-MMP (such as MT1-MMP) whereby an increase or decrease in the production amount, etc. of the MT-MMP (such as MT1-MMP) is compared to evaluate the efficacy of each additive. The additive subjected to the said screening method has no limitation but includes any species covering either known or novel substances so far as it is a candidate for a substance having a function of promoting or inhibiting the expression of MT-MMP (such as MT1-MMP). The said additive may include those which relate to the expression control of genes, antisense nucleic acids against MT-MMP (such as MT1-MMP), such as antisense RNA against MT-MMP (such as MT1-MMP) and ribozymes which cleaves MT-MMP mRNA (such as MT1-MMP mRNA) in a sequence-depending manner and others. The additive may be added to the culture liquid either directly or after being haptenized. A gene which expresses each of them may be introduced into cells. A quantitative immunoassay for MT-MMP (such as MT1-MMP) is valuable for designing and selecting nucleic acids (such as antisense RNA) and ribozymes which strongly and selectively suppress the expression of MT-MMP (such as MT1-MMP). MT-MMP inhibitors (such as MT1-MMP inhibitors) are useful as pharmaceutical agents concerning suppression of cancer metastasis.

The quantitative immunoassay for MT-MMP (such as MT1-MMP) according to the present invention is useful for tests in connection with cancer or cancer metastasis and also for tests in connection with the progress degree of rheumatoid arthritis and Alzheimer's disease. There are provided an agent for the test of cancer or cancer metastasis and also for the test of rheumatoid arthritis and Alzheimer's disease progress degrees. Particularly, a reagent for immunological quantitating test of MT-MMP (such as MT1-MMP) serves usefully as an agent for the test of cancer or cancer metastasis and also for the test of rheumatoid arthritis and Alzheimer's disease progress levels.

In the present invention, there is provided solid-phase anti-MT-MMP Ab such as solid-phase anti-MT1-MMP Ab. Particularly, solid-phase anti-MT-MMP mAb such as solid-phase anti-MT1-MMP mAb is provided. It is possible to screen for the enzymatic activity of MT1-MMP in a sample with solid-phase anti-MT-MMP Ab such as solid-phase anti-MT1-MMP Ab (for example, solid-phase monoclonal anti-MT1-MMP Ab). The said screening method is carried out, for example, in such a manner that an MT-MMP-containing sample such as an MT1-MMP-containing sample is contacted with anti-MT-MMP Ab such as anti-MT1-MMP Ab (for example, solid-phase monoclonal anti-MT1-MMP Ab) immobilized on a reactor, the MT-MMP is captured by the solid-phase anti-MT-MMP Ab (e.g., MT1-MMP is captured with the said anti-MT1-MMP Ab by means of antigen-antibody interaction) and then the enzymatic activity of MT-MMP such as the enzymatic activity of MT1-MMP is measured. Enzymatic activity measurement may be carried out, for example, with a substrate for MT-MMP (such as a substrate for MT1-MMP) by detecting or measuring a signal resulted by the enzymatic reaction. Particularly, a soluble form MT-MMP can be quantitated. Thus, it is possible to determine the correlation between the resulting soluble form MT-MMP amount and tumor malignancy or cancer metastatic potential. In another aspect of the present invention, there is provided immobilized MT-MMP such as immobilized MT1-MMP. There is particularly provided MT1-MMP which is solid-phased by an immunological method. The said solid-phase MT-MMP such as solid-phase MT1-MMP is contacted, for example, with a substrate for MT-MMP (such as a substrate for MT1-MMP) in the presence or absence of a sample (including MT-MMP activity inhibitors or other MT-MMP activity inhibitors) and signals produced as a result of the enzymatic reaction are detected or measured whereupon solid-phase MT-MMP can be used for screening. A typical solid-phase MT-MMP is attached to a solid phase on a reactor.

It is apparent that, instead of MT1-MMP, the above is adaptable similarly for MT-MMPs such as MT1-MMP, MT3-MMP, MT4-MMP, MT5-MMP and MT6-MMP.

Thus, in accordance with the present invention, it is now possible to measure the activity of MT-MMP and to search for inhibitors for MT-MMP activity. Particularly in the present invention, it is possible to measure the activity of MT1-MMP and to search for inhibitors for MT1-MMP activity. In the aforementioned method, MT-MMP (such as MT1-MMP) in the sample is specifically bound to a solid phase to search for its activity. There are also provided screening reagents therefor. It is further possible to evaluate the invading or metastatic potential of cancers from the resulting MT-MMP activity (such as MT1-MMP activity). Accordingly, cancer tissue samples can be assessed for their invading potential. There are also provided reagents used therefor. They are also useful for the selection of pharmaceutical drugs or agents and in the search for agents whereby variations in the invading potential of treated cancer tissues will be predicted and MT1-MMP production will be suppressed to reduce or extinguish the invading ability. In another aspect, the above-mentioned method enables assays for MT1-MMP-inhibiting activity wherein standard MT1-MMP having a predetermined activity level is used followed by adding various enzymatic activity inhibitors, MMPs inhibitors or any compound. When such a method is used, it is possible to search for unknown MT1-MMP inhibitors and to know an MT1-MMP inhibiting ability owned by the known MMP activity inhibitors including Batimastat, Marimastat, Ro32-3555, AG3340, Bayl2-9566, CGS27023A, etc. (which may include hydroxamic acid, carboxylic acid, phosphonic acid, thiol derivatives, etc. and are disclosed in, for example, WO 97/27174, A; WO 97/20824, A; GB 2268934 A or US Patent No. 5,310,763 A; US Patent No. 5,268,384 A; US Patent No. 5,455,258; WO 96/11209 A; WO 97/18207 A; WO 97/32846 A; WO 98/17643 A; JP, A, 7-101925 (1995); US Patent No. 5,614,625 A; WO 98/30551 A; WO 98/43963 A; WO 96/15096 A; WO 96/33174 A; WO 96/33968 A; WO 99/31052 A; International Application PCT/JP00/03166; etc.). It is obvious that the above can be similarly carried out for MT-MMP such as MT2-MMP, MT3-MMP, MT4-MMP, MT5-MMP and MT6-MMP as well instead of MT1-MMP.

Enzymatic activity- or enzyme-inhibiting activity-measurements can be carried out according to usual measuring methods. It is also possible to use various kinds of labels, buffer systems and other appropriate reagents. In conducting the method, it is possible that MT-MMPs are treated with an activator such as mercury aminophenylacetate or a MT-MMP precursor or a latent form MT-MMP is converted to an active form in advance.

For individual assays, suitable assay systems may be constructed by adaptations of technical consideration ordinarily given by artisans in the art over general conditions and operations suited to each of the methods.

For details of those conventional technical methods, a variety of reviews, texts, books, etc. may be referred to. They are, for example, Methods in Enzymology, Vol. 1 & 2 (Preparation and Assay of Enzymes), Academic Press, New York (1955); Methods in Enzymology, Vol. 5 (Preparation and Assay of Enzymes), Academic Press, New York (1962); Methods in Enzymology, Vol. 6 (Preparation and Assay of Enzymes), Academic Press, New York (1963); Methods in Enzymology, Vol. 3 (Preparation and Assay of Substrates), Academic Press, New York (1957); Methods in Enzymology, Vol. 4 (Special Techniques for the Enzymologist), Academic Press, New York (1957); Methods in Enzymology, Vol. 19 (Proteolytic Enzymes), Academic Press, New York (1970); Methods in Enzymology, Vol. 45 (Proteolytic Enzymes, Part B) Academic Press, New York (1976); Methods in Enzymology, Vol. 80 (Proteolytic Enzymes, Part C) Academic Press, New York (1982); Methods in Enzymology, Vol. 248 (Proteolytic Enzymes: Aspartic and Metallo Peptidases), Academic Press, New York (1995); etc. and references quoted in the above documents, the disclosures of which are incorporated herein by reference.

Reagents, reagent kits, and further kits (screening kits) which are useful in the assays (measuring methods) and the screening methods of the present invention are valuable. The reagent set or kit includes packs (and/or containers or packages) and kits (for example, acceptable in the assay fields including immunoassays or the pharmaceutical fields) comprising one or more containers filled with one or more of the components of the aforementioned compositions of the invention. Typically, associated with such a single or plural containers can be a notice (attached document) in the form prescribed by a governmental agency regulating the manufacture, use or sale of clinical tests, pharmaceuticals or biological products, reflecting approval by the agency of the manufacture, use or sale of the product for human administration.

When employed as pharmaceutical agents, the active components obtained or identified by the screening method according to the present invention (e.g., (a) compounds, or salts thereof, which promote or inhibit the expression of MT-MMP such as MT1-MMP, (b) compounds, or salts thereof, which promote or inhibit the enzymatic activity of MT-MMP such as MT1-MMP, etc,) may be administered usually in the form of a pharmaceutical composition or preparation alone or in admixture with a variety of pharmaceutically acceptable aids. Preferably they may be administered in the form of a convenient pharmaceutical composition or formulation suitable for oral, topical, parenteral application, or the like. Any of dosage forms (including those for inhalation and rectal administration) may be selected depending on purpose.

The active components of the present invention can also be used in admixture with anti-tumor agents (antineoplastic agents), tumor-metastasis inhibitors, anti-inflammatory agents and/or immunosuppressants. Any of the anti-tumor agents (antineoplastic agents), tumor-metastasis inhibitors, anti-inflammatory agents and/or immunosuppressants can be used without any limitation so long as they advantageously serve. Examples of such drugs are selected from drugs known in the art.

The pharmaceutical compositions can be formulated in accordance with conventional techniques. For example, the compound (active component) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, etc., or suitably in a combination thereof, depending on necessity, to give a unit dose form which is required for generally approved pharmaceutical practices.

Dose levels of said active component according to the present invention may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy, or other factors.

For the manufacture of pharmaceutical compositions and preparations, the additives, etc., preparation methods and the like can be suitably selected, depending on necessity, from those disclosed in Nippon Yakkyokuho Kaisetsusho Henshu Iinkai (Ed.), "14th Edition Nippon Yakkyokuho Kaisetsusho (Commentary on The Pharmacopoeia of Japan, 14th Edition)", June 27, 2001, Hirokawa Pub. Co., Tokyo, Japan; Hisashi Ichibagase et al. (Ed.), "Pharmaceutical Research and Development (Ikuo Suzuki, chief editor), Vol. 12 (Pharmaceutical Necessities 1)", October 15, 1990, Hirokawa Pub. Co., Tokyo, Japan; ibid., Vol. 12 (Pharmaceutical Necessities 2), October 28, 1990, Hirokawa Pub. Co., Tokyo, Japan; etc., all the disclosures of which are incorporated herein by reference.

For terms (words) and/or abbreviations used in the specification and in the drawings, they must conform with an "IUPAC-IUB Commission on Biochemical Nomenclature" or are based on the meanings of the terms which are commonly used in the art. Abbreviations as listed below are principally used hereinbelow:
bp: base pair(s);
IPTG: isopropyl-1-thio-β-D-galactopyranoside;
SDS: sodium dodecyl sulfate;
LB: Luria-Bertani
TMB: 3,3',5,5'-tetramethylbenzidine
BSA: bovine serum albumin
ELISA: enzyme-linked immunosorbent assay
HRP: horseradish peroxidase
PAGE: polyacrylamide gel electrophoresis
EDTA: ethylenediaminetetraacetic acid

The monoclonal anti-MT1-MMP antibody-producing hybridoma, designated 222-1D8, obtained in Example 2 (Table 1) mentioned herein below has been deposited as from October 26, 2000 (original deposit date) with International Patent Organism Depositary (IPOD), the National Institute of Advanced Industrial Science and Technology (AIST, an Independent Administrative Institution (IAI) under the Ministry of Economy, Trade and Industry (METI)), located at AIST Tsukuba Central 6, 1-1, Higashi 1-chome, Tsukuba-shi, IBARAKI (Zip Code: 305-8566), JAPAN (former name: the National Institute of Bioscience and Human Technology (NIBH), Agency of Industrial Science and Technology, Ministry of International Trade and Industry, at (former address) 1-3, Higashi 1-chome, Tsukuba-shi, IBARAKI (Zip Code: 305-8566), JAPAN) and has been assigned the Accession Number FERM P-18085. The original deposit of the hybridoma 222-1D8 has been transferred to one under the Budapest Treaty by a request dated November 12, 2001 and is on deposit with the Accession Number FERM BP-7795 under the terms of the Budapest Treaty at IPOD. Similarly, the monoclonal anti-MT1-MMP Ab-producing hybridoma, designated 222-2D12, obtained in Example 2 (Table 1) mentioned herein below has also been deposited as from October 26, 2000 (original deposit date) with IPOD and has been assigned the Accession Number FERM P-18086. The original deposit of the hybridoma 222-2D12 has been transferred to one under the Budapest Treaty by a request dated November 12, 2001 and is on deposit with the Accession Number FERM BP-7796 under the terms of the Budapest Treaty at IPOD.

### Examples

The present invention is specifically described by means of the following Examples which are provided only for illustrative purposes, and reference to specific embodiments of the present invention. Although these illustrative examples are provided for disclosing particular embodiments of the present invention, they should not be construed as limiting or restricting the scope of the present invention disclosed herein. It should be understood that various modes will be practicable based on the spirit of the present invention.

All the examples were or can be practiced using standard techniques well or conventionally known to those of ordinary skill in the art unless otherwise specified. For vectors, restriction enzymes, etc., commercially available products from Takara Shuzo K.K., Japan and others were used.

### Example 1

### Production of Recombinant MT1-MMP

Various recombinant MT1-MMP proteins were prepared and employed for immunization, screening for monoclonal antibodies, Western blotting, ELISA, etc.

### ① Recombinant Δ MT1-MMP (immunizing antigen) from Escherichia coli (E. coli)

### a) Construction of expression vector

The animal cell expression vector, pSGΔ MT1-MMP (prepared by the method disclosed in J. Cao et al., J. Biol. Chem., 270: 801-805 (1995)) was cut with with restriction enzymes Smal and BgIII to give the Δ MT1-MMP gene which was then ligated in-frame to β-galactosidase of pUC18 cleaved with restriction enzymes SmaI and BamHI to construct an expression vector pUCΔ MT1-MMP. Using the expression vector, a fusion protein with a molecular weight of about 55 kD was expressed, which fusion protein is comprised of total 499 amino acid residues consisting of 11 amino acid residues of β-galactosidase and 488 amino acid residues of Δ MT1-MMP (G⁴⁸ to G⁵³⁵) which lacks the signal peptide and part of the propeptide region (47 amino acid residues). The DNA coding for MT1-MMP is disclosed in, for example, J. Cao et al., J. Biol. Chem., 270: 801-805 (1995); K. Imai et al., Cancer Research, 56: 2707-2710 (1996); etc., the disclosures of which are incorporated hereby by reference.

### b) Expression of fusion proteins

E. coli DH5α carrying pUCΔ MT1-MMP was inoculated to an LB medium (4 mL) containing ampicillin (50 µg/mL) and incubated at 37°C for 16 hours to form a primary culture. This culture was re-inoculated to an LB medium (400 mL) containing ampicillin (50 µg/mL) and incubated at 37°C for about 3 hours until the middle stage of the exponential growth period. IPTG (final concentration: 0.1 mM) was added to the culture followed by continuing the incubation for another 4 hours to induce the expression of recombinant fusion proteins.

### c) Recombinant product purification-1

### (Inclusion body recovery and solubilization)

The culture was immediately cooled with ice and subjected to centrifugation (5,000 rpm, 20 min) to recover cells which were then suspended in 20 mM Tris-HCl (pH 8.0, 20 mL) containing lysozyme (1 mg/mL) and placed on ice for 15 min to destroy cell walls. The cell suspension was subjected to an ultrasonic disruption (30 sec, 10 times) with ice-cooling and centrifuged (18,000 rpm, 10 min) to recover inclusion bodies. The inclusion bodies were re-suspended in 10 mM phosphate buffer (pH 7.0, 1 mL) containing 0.1 M NaCl, washed and centrifuged (15,000 rpm, 10 min) to recover precipitates. The precipitate was dissolved in 10 mM phosphate buffer (pH 7.0, 1 mL) containing 8M urea and 0.1 M NaCl.

### d) Recombinant product purification-2

### (Preparative SDS-PAGE and electric elution)

To the solubilized fraction prepared in the above step was added 6 × SDS-PAGE sample buffer (reduction) at a 1/5 volume ratio and allowed to stand at 37°C for 1 hour. The mixture was then subjected to preparative SDS-PAGE. The samples were applied to gels at 0.6 mL per 90 × 70 × 1 mm gel. After completion of migration, the gel was dipped in 3M KC1, and protein bands were visualized. The recombinant Δ MT1-MMP band was cut out. The cut-out gel was sliced, washed with an SDS-PAGE migration buffer, placed in an electric elution column and applied with electricity of 200 volts (constant voltage) for 4 hours whereupon the recombinant protein was recovered. To the recombinant protein was added deoxycholic acid (DOC) so as to make its final DOC concentration 10 mM, and the mixture was allowed to stand in a cold room for 16 hours wherein SDS was substituted with DOC. The mixture was dialyzed against 50 mM phosphate buffer (pH 7.0) containing 0.1M NaCl whereupon excessive SDS and DOC were removed. Precipitates generated during the dialysis were removed by centrifugation and the resultant supernatant was concentrated by means of ultrafiltration. The soluble recombinant Δ MT1-MMP (Lot GV01) obtained from the E. coli culture (400 mL) was 2.6 mg (2 mg/mL × 1.3 mL).

### ②Recombinant MT-MMPs from COS-7

COS-7 cells were transfected with MT1-MMP cDNA inserted into pSG5 by the calcium phosphate method. After expression for 48 hours, the cells were harvested. The cells were washed with PBS, re-suspended in PBS (0.5 mL) and subjected to an ultrasonic disruption. To the resulting cell disintegrates was added 6 × SDS-PAGE sample buffer (containing a reducing agent) at a 1/5 volume ratio to afford an antigen sample for SDS-PAGE and western blotting. Similar treatments were carried out for each of MT2-MMP cDNA (mouse) inserted to pSG5 and MT3-MMP cDNA inserted to pSG5 to afford antigen samples. They were used in investigation for the cross reactivity of monoclonal antibodies.

### ③ Refolded recombinant Δ MT1-MMP (Δ MT1-MMPRF) from E. coli

pUCΔ MT1-MMP constructed in Example 1 was cut with restriction enzymes KpnI and HindIII to afford the Δ MT1-MMP gene which was ligated in-frame to pTrcHis to produce an expression vector pTrcHisΔ MT1-MMP. E. coli was transformed with this expression vector, followed by expression of recombinant proteins.

Insoluble inclusion bodies prepared from the above transformed E. coli culture (400 mL) were solubilized with 50 mM phosphate buffer (pH 7.5) containing 8 M urea, 0.5 M NaCl and 10 mM imidazole, and subjected to an Ni ⁺⁻ Chelating Sepharose (Pharmacia) gel column. After washing, the column was eluted with 50 mM phosphate buffer (pH 7.5) containing 8 M urea, 0.5 M NaCl and 0.5 M imidazole and the resulting purified fraction was subjected to a PD-10 (Pharmacia) gel column equilibrated with 20 mM Tris-HCl (pH 8.6) containing 8M urea and 0.3 M NaCl to exchange the buffer. To this were added 50 µM ZnSO₄ and 1 mM DTT, and A₂₈₀ was monitored. The protein solution was diluted with 20 mM Tris-HCl (pH 8.6) containing 8 M urea and 50 µM DTT whereby A₂₈₀ was made 0.1. To this was added 20 mM cysteamin to prepare a sample (50 mL) for refolding. This was dialyzed against 50 mM Tris-HCl (pH 8.6, 5 L) containing 0.15 M NaCl, 5 mM CaCl₂, 100 µM ZnSO₄, 5 mM 2-mercaptoethanol, 1 mM hydroxyethyl disulfide and 0.02% NaN₃ and then dialyzed against 50 mM Tris-HCl (pH 8.6) containing 0.15 M NaCl, 5 mM CaCl₂, 50 µM ZnSO₄ and 0.02% NaN₃ to refold the recombinant protein.

After the dialysis, the sample was centrifuged to remove insolubilized proteins and the protein amount was estimated by measuring A₂₈₀. The amount of the resulting Δ MT1-MMP was about 1.6 mg. Then, an aliquot of the Δ MT1-MMP sample was treated with trypsin (0.1 µg/mL) at 37°C for 1 hour. When the product was analyzed with SDS-PAGE, there were detected the following bands: Δ M1-MMP (580 kDa), hemopexin domain (30 and 34 kDa) and catalytic domain (21 kDa). This was concentrated and used as antigen for evaluation on ELISA and epitope mapping.

### Example 2

### Production and Selection of Antibodies

### ① Schedule for immunization

- Immunized animals:: female six-week-old BALB/c (2 animals)
- Purified antigen:: 50 mM phosphate buffer (pH 7.0) containing 2 mg/ml Δ MT1-MMP (obtained in Example 1① -d) and 0.1 mM NaCl
- Initial immunization:: Intraperitoneal administration at 58 µg/290 µl/mouse (complete Freund adjuvant)
- Additional immunization:: 20 days later, intraperitoneal administration at 64 µg/160 µl /mouse
- Additional immunization:: 35 days later, intraperitoneal administration at 54 µg/160 µl/mouse
- Final immunization:: 69 days later, intravenous administration at 73.3 µg/160 µl/mouse

Cell fusion was conducted 3 days later from the final immunization (72 days later)

Thus, the above purified Δ MT1-MMP antigen (58 µg/290 µl) was intraperitoneally administered to female 6-week-old BALB/c mice together with a complete Freund adjuvant for the initial immunization. Twenty days later, the above purified antigen (64 µg/160 µl) was intraperitoneally administered to the initially immunized mice for the additional immunization. Thirty five days later, the above purified antigen (54 µg/160 µl) was intraperitoneally administered for the second additional immunization. Sixty nine days later, the above purified antigen (73.3 µg/160 µl) was intravenously administered for the final immunization. Three days later after the final immunization (totally 76 days later), spleens were taken out from the immunized mice to prepare spleen cell suspensions.

### ② Cell fusion and hybridoma screening

For cell fusion, the following materials and methods were used:

RPMI-1640 medium: To RPMI-1640 (Flow Lab.) were added sodium bicarbonate (24 mM), sodium pyruvate (1 mM), penicillin G potassium (50 U/ml) and amikacin sulfate (100 µg/ml), and the mixture was adjusted pH to 7.2 with dry ice, sterilized and filtered through a 0.2 µm Toyo membrane filter.

NS-1 medium: To the above RPMI-1640 medium was added filter sterilized fetal calf serum (FCS; M. A. Bioproducts) until a concentration of FCS reached to 15% (v/v).

PEG-4000 solution: To RPMI-1640 medium was added polyethylene glycol 4000 (PEG-4000, Merck & Co.) until a concentration of PEG 4000 reached 50% (w/w). Thus, the serum-free solution was prepared.

Cell fusion using 8-azaguanine-resistant myeloma SP-2 cells (SP-2/0-Ag14 was carried out by slightly modified methods according to Oi & Herzenberg techniques disclosed in "Selected Method in cellular immunology, pp.351 to 372 (ed. B. B. Mishell and S. N. Shiigi), W. H. Freeman and Company (1980)".

The respective nucleated spleen cells (viable cell ratio: 100%) prepared in the foregoing were fused with myeloma cells (viable cell ratio: 100%) at a ratio of 5:1 to 10:1 according to the following procedure:

Each purified Δ MT1-MMP antigen-immunized spleen cell suspension and the myeloma cells were washed respectively with an RPMI 1640 medium followed by resuspending in the same medium. For fusion, the nucleated spleen cells and the myeloma cells were mixed together.

The cell suspension was then precipitated by centrifugation and the supernatant was completely aspirated off. To the cell pellet was added a PEG 4000 solution (RPMI 1640 medium containing 50% (w/v) polyethylene glycol 4000) pre-warmed to 37°C dropwise for 1 min (the volume of the PEG 4000 solution to be added was determined to give about 10⁷ myeloma cells/mL), and stirred for 1 min to allow the cells to be resuspended and dispersed. Next, 37°C pre-warmed RPMI 1640 medium was added dropwise for 2 minutes (RPMI 1640 medium : 50% PEG 4000-containing RPMI 1640 medium already added = 2 : 1 in volume). Further, RPMI 1640 medium was added dropwise within 2 to 3 minutes with stirring (RPMI 1640 medium : 50% PEG 4000-containing RPMI 1640 medium = 7 : 1 in volume) to allow the cells to be dispersed. This cell dispersion was centrifuged, and the supernatant fluid was completely aspirated off. To the cell pellet was added 37°C pre-warmed NS-1 medium quickly until a concentration of myeloma cells reached about 10⁶ cells/mL, and a large cell mass was carefully dispersed by pipetting. Next, the cell suspension was diluted with the same medium. The cells were plated on each well of a polystyrene 96-well microtiter tray (the number of plated myeloma cells per well was adjusted). The cell-containing microwell was incubated at 37°C under a 100% humidified atmosphere containing 7% CO₂/93% air.

Next, hybridoma cells were selectively grown in a selection medium.
(1) Media to be used were as follows:
   HAT medium: To NS-1 medium as described in the foregoing was added further hypoxanthine (100 µM), aminopterin (0.4 µM), and thymidine (16 µM).
   HT medium: The medium has the same composition as the foregoing HAT medium except that aminopterin was excluded.
(2) Next day (1st day) from culture initiation of the foregoing, two drops of HAT medium (about 0.1 mL) was added to the cells with a Pasteur pipette. On the 2nd, 3rd, 5th, and 8th days, a half of the medium (about 0.1 ml) was replaced with fresh HAT medium, respectively. On the 10th day, a half of the medium was replaced with fresh HT medium. On the 14th day or later, positive wells were examined by solid phase-antibody binding test (enzyme-linked immunosorbent assay; ELISA) for all wells wherein the growth of hybridomas was visually recognized.

Polystyrene 96-well plates were coated with each immunizing antigen at 100ng/well wherein each well was washed with PBS containing 0.05% Tween 20 to remove unadsorbed antigen proteins. To each antigen-coated well was added a supernatant fluid from the hybridoma well in which hybridomas were grown and the antigen-coated well was allowed to stand at room temperature for approximately one hour. After washing, horseradish peroxidase (HRP)-labeled goat anti-mouse immunoglobulin (Cappel Lab.) was added as a second antibody to the antigen-coated well, and the well was further allowed to stand at room temperature for approximately 1 hour. Next, after washing, to the well was added substrates, hydrogen peroxide and 3,3',5,5'-tetramethylbenzidine (TMB), and OD readings at 450 nm were obtained by a microplate OD reader (MRP-A4, Toso). Fifteen clones were selected.

### ③ Cloning of Hybridomas

Hybridomas in the wells positive against each immunizing antigen obtained in the foregoing were cloned by limiting dilution to establish monoclones.

That is, a cloning medium containing, as feeder cells, mouse thymocytes was prepared. Into a 96-well microtiter tray was plated hybridomas at a cell density of 5, 1, or 0.5 cells per well, respectively, with dilutions wherein the 5, 1, or 0.5 hybridoma cells per well was plated to 36, 36, and 24 wells, respectively. On the 3th to 7th and 8th to 15th days, NS-1 medium was added to all the wells. Approximately 1 to 3 weeks later from the initiation of cloning, the aforementioned method (ELISA) was conducted for groups wherein the sufficient growth of hybridomas was visually recognized and the rate of colony formation-negative wells is 50% or more. In cases where all the examined wells were negative, 4 to 6 wells each containing 1 colony were selected from antibody-positive wells, and recloned. Finally, anti-immunizing antigen antibody-producing hybridoma cells were obtained. Fifteen clones were selected.

### ④Subclass assay for monoclonal antibodies

Each subclass was determined for the 15 clones. According to the above-mentioned ELISA, each hybridoma supernatant was added to immunizing antigen-coated polystyrene 96-well plates. Next, after PBS washing, iso-type specific rabbit anti-mouse IgG antibodies (Zymed Lab.) were added. After PBS washing, horseradish peroxidase-labeled goat anti-rabbit IgG (H+L) was added, and visualization was carried out with hydrogen peroxide and 2,2'-azino-di(3-ethylbenzo-thiazolinic acid). As a result, the class and sub-class were determined.

### ⑤Selection by immunoblotting

### Reactivity test-1

Immunizing antigens were developed by 12% SDS-PAGE and transferred to a Nylon membrane. Each hybridoma culture supernatant was added thereto as a primary antibody and anti-mouse Ig (G+A+M)-HRP was used as a secondary antibody for staining. Among all 15 clones, 8 clones were judged to be positive and subjected to the secondary selection. For subclasses with regard to those 8 clones, 4 clone were IgGl/K *,* 1 clone was IgG3/K and 3 clones were IgM.

### Reactivity test-2

The eight clones which were judged to be positive in the foregoing were further subjected to selection by Western blotting using transformant MT1-MMP-expressing COS-7 cell disintegrates as antigens whereupon weakly reactive 3 clones ((+) in Table 1) were excluded. All of them were IgM. Finally five clones were obtained where the subclass of 4 clones was IgG1/κ and that of one clone was IgG3/κ

### ⑥Hybridoma cultivation and purification of monoclonal antibodies

Each hybridoma cell thus obtained was grown in NS-1 medium to afford monoclonal antibodies with a concentration of 10 to 100 µg/ml in the culture supernatant.

Alternatively, 10⁷ hybridoma cells thus obtained were administered intraperitoneally to a mouse (inbred Balb/c mouse, ♀ , six-week old) primed intraperitoneally with Pristane 1 to 2 weeks prior to hybridoma injection, and 1 to 2 weeks later an ascites containing 4 to 7 mg/mL monoclonal antibody was recollected. After the obtained ascitic fluid was salted out with 40% ammonium sulfate saturation, IgG class antibodies were adsorbed on protein A affigel (Bio-Rad), followed by elution with 0.1 M citrate buffer, pH 5.0 to afford purified forms.

In the present invention, monoclonal antibody-producing hybridoma cells (5 clones) obtained in the foregoing were intraperitoneally administered to mice and ascitic fluids containing individual monoclonal antibodies were prepared. The resulting ascitic fluid was subjected to ammonium sulfate fractionation, and an affigel protein A gel column to give purified monoclonal antibodies.

Results for subclass assay, reactivity test-1 and reactivity test-2 are summarized in Table 1.

**Table 1**

| Clone No. | Subclass | Reactivity Test -1 | Reactivity Test - 2 |
|---|---|---|---|
| 222-1D8 | γ1/κ | + | + |
| 222-2D12 | γ1/κ | + | + |
| 222-3E12 | γ1/ κ | + | + |
| 222-4G5 | γ1/κ | + | + |
| 222-6D2 | µ/κ | + | (+) |
| 222-9A3 | γ3/κ | + | + |
| 222-10E6 | µ/κ | + | (+) |
| 222-14H6 | µ/κ | + | (+) |

### Example 3

### Reactivity of Antibodies

### ①Epitope mapping

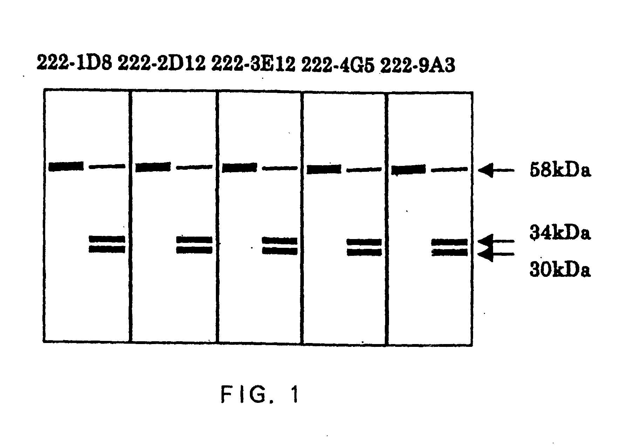

An epitope mapping was carried out by Western blotting using Δ MT1-MMP (58 kDa) prepared in Example 1-③) , as well as hemopexin domain (30 kDa, 34 kDa) and catalytic domain (21 kDa) fragments as antigens. The five clones selected in Example 2 were added as the primary antibodies and anti-mouse Ig (G+A+M)-HRP was used as the secondary antibody for staining. Any of the clones strongly reacted with the hemopexin domain. It was verified that those epitopes were positioned on the hemopexin (see Fig. 1).

### ② Cross reactivity

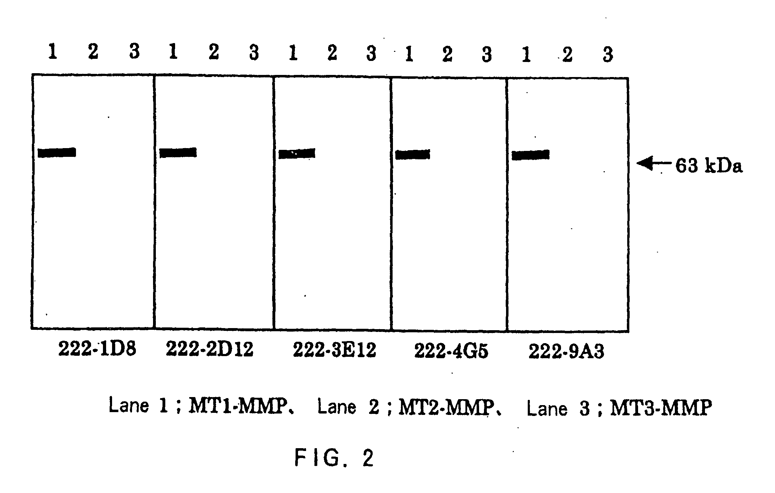

Cross reaction tests were carried out by Western blotting using, as antigens, MT1-MMP-expressing COS-7 cell disintegrates, MT2-MMP (mouse)-expressing COS-7 cell disintegrates and MT3-MMP-expressing COS 7 cell disintegrates as prepared in Example 1-②. The five clones selected in Example 2 were added as the primary antibodies and anti-mouse Ig (G+A+M)-HRP was used as the secondary antibody for staining. Any of the clones reacted only with MT1-MMP but did not react with MT2-MMP (mouse) and MT3-MMP. It was verified that they had a specificity to MT1-MMP (see Fig. 2).

### Example 4

### Assay for MT1-MMP

In a 96-well microplate where each antibody was solid-phased, standard antigens (recombinant Δ MT1-MMP) was made to react therewith. After washing, reaction was conducted with each antibody IgG-HPR and then unreacted IgG-HRP was removed by washing. IgG-HRP bound via the antigen was assayed with TMB as a substrate for its residual activity. A combination of solid-phase antibody 222-2D12 with labeled antibody 222-1D8, giving the best antigen detection performance, was found. Further, the concentration of used antibodies, reaction time, reaction temperature, reaction buffer compositions, etc. were fully investigated. For the labeled antibodies, Fab'-HRP was newly prepared and used in place of IgG-HRP. As a result, MT1-MMP was most effectively assayable in the following method:

### ①Labeled antibodies

Labeled antibody Fab'-HRP was prepared as follows:

For example, monoclonal antibodies were digested with pepsin (enzyme: antibody ratio, 2% w/w) in 0.1M sodium acetate buffer, pH4.2 containing 0.1M NaCl at 37°C for 20 hrs. The digestion was stopped with the addition of 3M Tris-HCl buffer, pH7.5. F(ab')₂ fractions were collected by gel filtration on an Ultrogel AcA54 column equilibrated with 0.1M phosphate buffer, pH7.0. Cysteamine hydrochloride was then added to the F(ab' )2 fractions until the final concentration reached to 0.01M. The fragments were reduced at 37°C for 1.5 hr. Fab' fractions were collected by gel filtration on an Ultrogel AcA54 column equilibrated with 0.1M phosphate buffer, pH6.0 containing 5mM EDTA.

In another procedure, HRP was dissolved in 0.1 M phosphate buffer, pH7.0, followed by addition of EMCS in DMF (EMCS:HRP molar ratio, 25:1). The mixture was incubated at 30°C for 30 min. and subjected to gel filtration on an NICK-5 column (Pharmacia) equilibrated with 0.1 M phosphate buffer, pH6.0 to collect maleimide-conjugated HRP fractions.

The Fab' fraction was mixed with the maleimide-conjugated HRP fraction to form an equimolar mixture. The resultant mixture was incubated at 4°C for 20 hrs, followed by blocking of unreacted thiol groups with N-ethyl maleimide (N-ethyl maleimide:Fab' molar ratio, 10:1). The mixture was subjected to gel filtration on an Ultrogel AcA54 column equilibrated with 0.1M phosphate buffer, pH6.5 to collect labeled antibodies, Fab'-HRP conjugates. The labeled antibody fraction supplemented with 0.1% BSA and 0.001% chlorhexidine was stored at 4°C.

### ② Antibody-solid phased plates

Monoclonal antibodies (e.g., 333-2D12 (IgG)) were dissolved in 0.1M phosphate buffer, pH 7.0 to a concentration of 25 µg/mL. The monoclonal antibody solution was added to each well of a 96-well microwell (Maxisorp; Nunc-Immuno Module) at 100 µL per well and allowed to stand at 4°C for 24 hrs. After removal of the monoclonal antibody solution, each well is rinsed three times with a washing liquid (10 mM phosphate buffer, pH 7.0, containing 0.1% Tween 20 and 0.1 M NaCl), followed by the addition of a blocking liquid (30 mM phosphate buffer, pH 7.0, containing 1% BSA, 10 mM EDTA and 0.1 M NaCl) at 0.3 mL per well. The solid-phase antibodies were preserved at 4°C.

### ④ Standard antigens

Δ MT1-MMP purified in Example 1 was dissolved in a dilution buffer (30 mM phosphate buffer, pH 7.0, containing 0.45% SDS, 0.4% 2-mercaptoethanol, 3% horse serum, 1% BSA, 10 mM EDTA and 0.1 M NaCl) to a concentration of 160 ng/mL. This antigen solution was diluted with a dilution buffer to give standard antigen dilutions with a series of the following antigen concentrations: 80, 40, 20, 10, 5 and 2.5 ng/mL.

### ④Samples to be assayed

Samples to be assayed were diluted with a dilution buffer upon necessity.

### ⑤Primary reaction

Standard antigens or samples to be assayed were dispensed into a 96-well vinyl plate at 25 µL each. To each well was added a dilution buffer in aliquots of 100 µL, followed by mixing. An aliquot (100 µL) of the resultant mixture was taken out, dispensed into an antibody-solid phased plate from which a blocking solution was removed by washing. The plate was then allowed to stand at 4°C for 16 hours.

### ⑥Secondary reaction

Standard antibody 222-1D8 (Fab'-HRP) was diluted with a blocking solution to a concentration of 0.4 µg/mL. The reaction solution was removed from the antibody-solid phased plate where the primary reaction was completed. The plate was washed with a washing liquid three times and an aliquot (100 µL) of the labeled antibody solution was added to each well. The plate was allowed to stand at room temperature for 1 hour.

### ⑦ Visualization (Coloration)

After completion of the reaction, the labeled antibody solution was removed. The plate was then washed with a washing liquid three times, and a TMB solution (CALBIOCHEM) was added to each well at 100 µL. The mixture was allowed to react at room temperature for 20 minute. The reaction was stopped with the addition of 2N sulfuric acid at 100 µL per well. The reaction mixture was measured for A₄₅₀ with a microplate reader (MPR-A4; Tosoh).

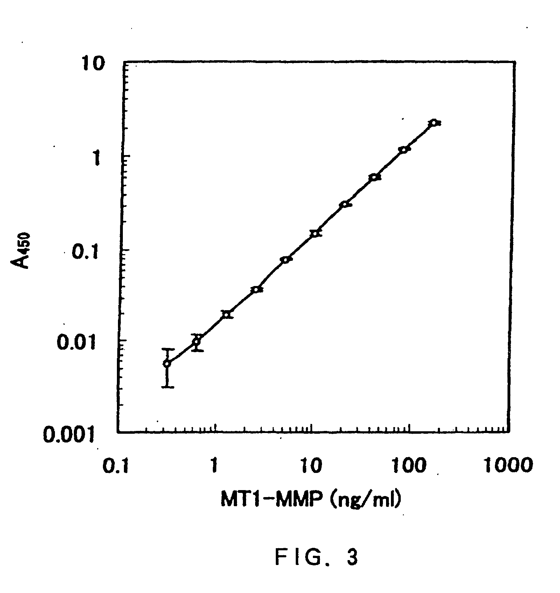

An example of the standard curves obtained by the above method is shown in Fig. 3. It has been noted that MT1-MMP sandwich EIA has a sensitivity of 0.32 ng/mL (6.4 pg/well) and a linear correlation within a range of 0.63 to 160 ng/mL (0.013 to 3.2 ng/well).

### Example 5

### MT1-MMP sandwich EIA Performance

### ① Determination of sample amounts by dilution tests

Assay samples prepared by addition of standard antigens to each of serum and synoval fluid were diluted and subjected to assays according to the method disclosed in Example 4. The serum sample was diluted to give sample dilutions with a series of the following concentrations: 10, 20 and 30 µL/well, and assayed. When the sample amount was made 20 µL/well or less, the added standard antigen was recovered 90% or more. Therefore, 20 µL/well was set for the serum sample assay. The synoval fluid sample was diluted to give sample dilutions with a series of the following concentrations: 0.625, 1.25, 2.5 and 5u µL/well and assayed. When the sample amount was 2.5 µL/well, the added standard antigen was recovered 80% or more. Therefore, 2.5 µL/well was set for the synoval fluid sample (Table 2).

**Table 2**

| Sample (Amount of Standard Substance Added) | Amount of Sample Added (µL/well) | Measured Value (ng/mL) | Recovery Rate (%) |
|---|---|---|---|
| Serum 1(40ng/mL) | 10 | 39.4 | 98.5 |
| | 20 | 37.4 | 93.5 |
| | | | |
| Serum 2(10n9/mL) | 10 | 10.3 | 103.0 |
| | 20 | 9.6 | 96.0 |
| | | | |
| Serum 3(2.5ng/mL) | 10 | 2.7 | 108.0 |
| | 20 | 2.4 | 96.0 |
| | | | |
| Articular Liquid 1 ( 142ng/mL ) | 0.625 | 155.8 | 109.7 |
| | 1.25 | 143.6 | 101.1 |
| | 2.5 | 127.2 | 89.6 |
| | | | |
| Articular Liquid 2(142ng/mL) | 0.625 | 154.2 | 108.6 |
| | 1.25 | 138.3 | 97.4 |
| | 2.5 | 123.5 | 87.0 |

### ② Admixture-Recovery Test

To each of serum, synoval fluid, culture supernatant (Con A-treated MDA-MB-231) and cell extract fraction (Con A-treated MDA-MB-231) samples were added standard antigens and the admixed standard antigen in the samples was quantitated in the same manner as in Example 4. Each recovery rate of the added standard antigen was checked. An exampple of assay results is shown in Table 3. For the serum, culture supernatant and cell extract fraction samples, the recovery rate of not less than 90% was ensured. For the synoval fluid sample, the recovery rate of not less than 80% was ensured.

**Table 3**

| Samples | Amount of Antigen Added (ng/mL) | Amount of Antigen Recovered (ng/mL) | Recovery Rate (%) |
|---|---|---|---|
| Culture Supernatant | 40.0 | 42.3 | 105.8 |
| | 20.0 | 18.6 | 93.0 |
| | 10.0 | 10.3 | 103.0 |
| | | | |
| Cell Extract Fraction | 40.0 | 40.7 | 101.8 |
| | 20.0 | 18.0 | 90.0 |
| | 10.0 | 9.9 | 99.0 |

### ③ Simultaneous reproducibility

a) To sera were added standard antigens to prepare serum samples (3 samples). Each sample was assayed simultaneously (n = 8) in the same manner as in Example 4. The CV values were 2.5 to 3.4% (Table 4).

**Table 4**

| Sample Nos. | Measured Value ±SD | CV(%) |
|---|---|---|
| 1 | 69.1±2.3 ng/mL | 3.4 |
| 2 | 36.0±0.9 ng/mL | 2.5 |
| 3 | 17.6±0.6 ng/mL | 3.2 |

b) Con A-treated MDA-MB-231 cell culture supernatant and cell extract fraction samples were subjected to simultaneous assays (n = 6) according to the method as disclosed in in Example 4. The CV values were 6.9% and 9.5%, respectively (Table 5).

**Table 5**

| Samples | Measured Value ±SD | CV(%) |
|---|---|---|
| Culture Supernatant | 85.5±6.9 ng/mL | 6.9 |
| Cell Extract Fraction | 87.7±8.4 ng/mL | 9.5 |

### ④Variations among assays

a) To sera were added standard antigens to prepare serum samples (3 samples). Each sample was assayed 8 times in the same manner as in Example 4. The CV values were 3.1 to 5.0% (Table 6).

**Table 6**

| Sample Nos. | Measured Value ±SD | CV(%) |
|---|---|---|
| 1 | 67.5±2.2 ng/mL | 3.3 |
| 2 | 34.9±1.7 ng/mL | 5.0 |
| 3 | 18.0±0.6 ng/mL | 3.1 |

b) Each of Con A-treated MDA-MB-231 cell culture supernatant and cell extract fraction samples was assayed 6 times in the same manner as in Example 4. The CV values were 7.1% and 10.2%, respectively (Table 7).

**Table 7**

| Samples | Measured Value ±SD | CV(%) |
|---|---|---|
| Culture Supernatant | 87.7±6.3 ng/mL | 7.1 |
| Cell Extract Fraction | 92.6±9.5 ng/mL | 10.2 |

### ⑤ Specificity Tests

Each of MMP-1, -2, -3, -4, -7, -8, -9, -13, -19 and -20 was prepared to give a concentration of 1 µg/mL, and subjected to MT1-MMP sandwich EIA. A₄₅₀ was measured. The results are shown in in Table 8. When MT1-MMP was subjected to sandwich EIA at 0.16 µg/mL, the absorbance was 2.206. However, when the above MMPs were subjected, their absorbances were only an extent of 0.024 to 0.071. Accordingly, it has been verified that MT1-MMP sandwich EIA is specifically reactive with MT1-MMP.

**Table 8**

| Cross Reactivity on MT1-MMP Sandwich EIA | | |
|---|---|---|
| MMPs | concentration | A₄₅₀ |
| MMP-1 | 1 | 0.025 |
| MMP-2 | 1 | 0.024 |
| MMP-3 | 1 | 0.027 |
| MMP-7 | 1 | 0.026 |
| MMP-8 | 1 | 0.028 |
| MMP-9 | 1 | 0.039 |
| MMP-13 | 1 | 0.026 |
| MMP-19 | 1 | 0.027 |
| MMP-20 | 1 | 0.071 |
| MT1-MMP | 0.16 | 2.206 |
| Blank | - | 0.027 |

Sandwich EIA systems capable of specifically detecting and/or measuring MT1-MMP can be constructed by a combination of suitable antibodies where at least one member selected from monoclonal antibodies as prepared in the present invention is contained. The EIA systems may include either of one-step and two-step techniques but labeled antibodies are not limited to Fab'-HRP. Compositions for each reaction buffer, reaction conditions can be adjusted depending on various purposes of assaying. For example, the reaction time can be shortened or extended. Standard MT1-MMP samples can be obtained by isolation and purification from tissue culture supernatants, cell culture supernatants, and transformants or transfectants wherein the MT1-MMP is expressed by techniques as described in Example 1 or other methods. The purification can be conducted by a combination of ion-exchange chromatography, gel filtration, affinity chromatography using monoclonal antibodies, and/or other various affinity chromatographic means.

Samples to be tested are prepared from body fluid components derived from human being such as human blood, serum, plasma, synoval fluid, urine, saliva, cerebrospinal fluid and amniotic fluid, extracts from various human tissues (including various tumor and cancer tissues), culture cell extracts and supernatants of various cultured cells such as human derived transformants or transfectants, etc.

### Example 6

### Significance of Surfactants and Reducing Agents in MT1-MMP Sandwich EIA

COS-7 cells were transfected with Δ MT1-MMP gene by the method mentioned in Example 1 to produce Δ MT1-MMP-expressing cells. The COS-7 cell was cultured for 48 hours, and the culture supernatants were harvested. The cells in the culture supernatant were subjected to SDS-PAGE and Western blotting and it was verified that Δ MT1-MMP was present as a soluble molecule. When the culture supernatant was subject to MT1-MMP sandwich EIA, a sufficient signal (A₄₅₀ = 1.807) was detected. On the other hand, when the same culture supernatant was subjected to MT1-MMP sandwich EIA using an SDS and 2-mercaptoethanol-free dilution buffer (30 mM phosphate buffer, pH 7.0, containing 3% horse serum, 1% BSA, 10 mM EDTA and 0.1 M NaCl), signal lowered to an extent of about 1/4 (A₄₅₀ = 0.138; a table on the upper column of Table 9). It is likely that SDS and 2-mercaptoethanol acted on the antigen in the sample and a change in structure which is detectable by MT1-MMP sandwich EIA was resulted in the high-order structure of the antigen. In this structure change, the coexistence of SDS and 2-mercaptoethanol was essential (refer to the lower column in Table 9).

**Table 9**

| Condition for Conducting EIA | | A450 |
|---|---|---|
| Dilution Buffer containing Surfactant and Reducing Agent | | 1,807 |
| Dilution Buffer containing neither Surfactant nor Reducing Agent | | 0,138 |
| | | |
| Condition for Conducting EIA | | A450 |
| Surfactant | Reducing Agent | A450 |
| contained | contained | 1.163 |
| contained | not contained | 0.201 |
| not contained | contained | 0.027 |
| not contained | not contained | 0.040 |
| | | |

### Example 7

### Searching for MT1-MMP-Producing Culture Cell Lines

Searches for human MT1-MMP-producing culture cell lines were carried out by means of MT1-MMP sandwich EIA. With regard to the cell lines, there were used Burkitt's lymphoma (Daudi, Namalwa, Raji), acute lymphoblastic leukemia (T-cells) (MOLT-4), chronic myelogenous leukemia (K-562), promyelocytic leukemia (HL-60), fibrosarcoma (HT-1080), breast cancer (MDA-MB-231, SK-BR-3, MCF 7), lung cancer (A 549), rectal adenocarcinoma (SW 837), osteosarcoma (MG-33), lingual squamous cell carcinoma (SSC-25), uterocervical cancer (HeLa), cervical epidermoid cancer (CaSki), prostatic cancer (PC-3) and malignant melanoma (A-375) cells. Each of the cancer cell lines was incubated in a plate of 10 cm diameter until being confluent, the medium was exchanged with serum-free medium or Con A-containing serum-free medium and, after 48 hours, the culture supernatant and the cells were recovered. The cells were ultrasonically disrupted in 0.5 mL of dilution buffer and centrifuged. The resulting supernatant was subjected to EIA as a cell extract fraction. The culture supernatant was subjected to EIA as it was.

Examples of the measured data are shown in Table 10.

**Table 10**

| Cell Lines | | Culture Supernatant | | Celt Extract Fraction | |
|---|---|---|---|---|---|
| | | - Con A | +Con A | -Con A | +Con A |
| HT-1080 | Fibrosarcoma | 10.1 | 80.3 | 45.9 | 42.6 |
| MDA-MB-231 | Breast Cancer | 7.6 | 48.8 | 42.8 | 85.0 |
| SW 837 | Rectal Adenocarcinoma | 1.8 | 14.2 | 21.5 | 30.7 |
| MG-63 | Osteosarcoma | 1.6 | 20.3 | 0.7 | 0.0 |
| SSC-25 | Lingual Squamous Cell Carcinoma | 2.8 | 43.1 | 5.9 | 19.1 |
| CaSki | Cerevical Epidermoid Cancer | 1.7 | 25.1 | 9.0 | 9.5 |

MT1-MMP was detected in fibrosarcoma (HT 1080), a part of breast cancer (DMA-MB-231), rectal adenocarcinoma (SW 837), osteosarcoma (MG-63), lingual squamous cell cancer (SSC-25), cerevical epidermoid cancer (CaSki) cells, etc.

### Example 8

### Search for Factors, Pharmaceutical Drugs, etc. Modulating the Expressed Level of MT1-MMP

MT1-MMP-expressing culture cells are incubated after addition of physiologically active factors, pharmaceutical drugs or compounds, then the supernatants or cell extract fractions thereof are harvested, and subjected to MT1-MMP sandwich EIA. In the measurement, the product prepared from untreated culture cells is used as a control and increase or decrease in the production amounts of MT1-MMP are compared whereby the efficacy of each additive is evaluated. It is necessary that concentrations of the additive are appropriately adjusted. with regard to the additive, there may be exemplified those which relate to expression control of gene antisense RNA of MT1-MMP and ribozyme which cleaves mRNA of MT1-MMP in a sequence-dependently manner. The additive may be added to the culture solution either directly or after being haptenized or genes which expresses them each may be introduced into the cells. MT1-MMP sandwich EIA is useful for design and selection of ribozyme and antisense RNA which strongly and specifically suppress the expression of MT1-MMP. An inhibitor for the expression of MT1-MMP is useful as a pharmaceutical for suppression of cancer metastasis.

### Example 9

### Assay for MT1-MMP activity and Searching for MT1-MMP Activity Inhibitors

Anti-MT1-MMP monoclonal antibody was prepared into 25 µg/mL using 0.1 M phosphate buffer (pH 7.0), each 100 µL thereof were added to each 96-well microplate and allowed to stand at 4°C for 24 hours. Then the antibody solution was removed and washed with a washing liquid (10 mM phosphate buffer (pH 7.0) containing 0.1% Tween 20 and 0.1 M NaCl) for three times, 0.3 mL of a blocking solution (30 mM phosphate buffer (pH 7.0) containing 1% BSA, 10 mM EDTA and 0.1 M NaCI) was added to each 96-well microwell and the mixture was allowed to stand at 4°C for 16 hours or longer and preserved. This was washed with 10 mM phosphate buffer (pH 7.0) containing 0.1% Tween 20 and 0.1 M NaCl a sample containing MT1-MMP diluted with 30 mM phosphate buffer (pH 7.0) containing 1% BSA and 0.1 M NaCl was added thereto and the mixture was allowed to stand at 4°C for 16 hours. This was washed with 20 mM Tris-HCl buffer (pH 7.4) containing 0.1% Tween 20 and 0.15 M NaCl then 100 µL of Mca-Pro-Leu-Gly-Leu-Dpa-Ala-Arg-NH2 prepared into 1 µM with 50 mM Tris-HCl buffer (pH 7.5) containing 0.15 M NaCl 10 mM CaCl₂, 0.05% Brij 35 and 0.02% NaN₃ were added thereto and the mixture was allowed to stand at 37°C for 1 hour. The reaction solution was placed in a test tube to which 500 µL of 0.1 M sodium acetate buffer (pH 4.0) were added to stop the reaction. Degradation activity of the fluorescent substrate of MT1-MMP was measured in terms of fluorescent intensity (FI, Ex: 328 nm, Em: 393 nm) using a fluorophotometer RF-5000 (Shimadzu). The above-mentioned antibody concentration, type and concentration of the fluorescent substrate, composition of various reaction solutions, reaction time, reaction temperature, reaction solution volume, etc. may be appropriately changed if necessary.

### ① Assay for MT1-MMP activity

When various kinds of cancer tissue extract fractions were used as the samples to be tested in the above-mentioned method, it is possible that MT1-MMP was specifically made into solid phase from tissue extract fraction containing various kinds of MMPs and their activity was detected. From the resulting MT1-MMP activity, invasion ability of the cancer tissue sample can be estimated. It is also useful for the estimation of the variation of invasion ability of the treated cancer tissues or for the selection of drugs or for the search of the agents which suppress the production of MT1-MMP and lower or extinguish the invasion ability.

### ② Searching for inhibiting agents against MT1-MMP activity

When a standard MT1-MMP having a certain activity level of the sample added is used and various kinds of enzymatic activity inhibitors, MMPs inhibitors or compounds thereto are added in the above-mentioned method, it is now possible to measure the inhibiting activity to each of the MT1-MMPs. When this method is used, it is possible to search the unknown MT1-MMP inhibitors or to know the MT1-MMP inhibiting ability of the known MMP activity inhibitors such as Batimastat, Marimastat, Ro 32-3555, AG 3340, Bay 12-9566 and CGS 17023 A.

With regard to the standard MT1-MMP, it is possible to use recombinant protein expressed from E. coli into which pUCΔ MT1-MMP or pTrcHisΔ MT1-MMP described in Example 1 is introduced or recombinant protein expressed from COS-1, COS-7 or CHO cell into which pSGΔ MT1-MMP is introduced.

Prodomain of MT1-MMP was contained in pUCΔ MT1-MMP and pTrcHisΔ MT1-MMP and, when it is used as the standard MT1-MMP for the measurement of activity, it is necessary that the prodomain is cleaved by furin and removed so as to convert to MT1-MMP of an activated type. In order to directly express the activated form MT1-MMP by Escherichia coli expression, the following recombinants were prepared.

MT1-MMP cDNA was used as a template for amplification with primers: MT1ACTF1: GAAGATCTACGCCATCCAGGG (SEQ ID NO: 1) and MT1ACTR1 : GAGGTACCTCAGCCCATCCAGTCCC (SEQ ID NO: 2) and the amplified gene was cleaved by restriction enzymes BgIII and KpnI and ligated to pQE-32 (Qiagen) which was previously cleaved with BamHI and KpnI to give pQE MT1ACTR1.

Further, MT1-MMP cDNA was used as a template for amplification with primers: MT1ACTF1 : GAAGATCTACGCCATCCAGGG (SEQ ID NO: 1) and MT1ACTR2 : TAGGTACCTACCCGCCGCCCTCC (SEQ ID NO: 3) and the amplified gene was cleaved by restriction enzymes BgIII and KpnI and ligated to pQE-32 (Qiagen) which was previously cleaved with BamHI and KpnI to give pQE MT1ACTR2.

MT1-MMPs encoded by pQE MT1ACTR1 and pQE MT1ACTR2 are Y¹¹² to G⁵⁰⁷ and Y¹¹² to G⁵³⁵, respectively and both lack a prodomain and express activated form MT1-MMP.

Since pQE MT1ACTR1 lacks entire membrane-associating domain having a high hydrophobicity and pQE MT1ACTR2 lacks a part of the membrane-associating domain, solubility of the expressed recombinant protein is improved and has more favorable property as a standard MT1-MMP for the measurement of activity.

The recombinant activated form MT1-MMP can be purified by, for example, a method mentioned in Example 1-③ and used as a standard MT1-MMP for the measurement of activity.

### ③ Preparation of activated form MT1-MMP and activity assay

An insoluble inclusion body prepared from transformant E. coli (400 mL culture) into which pQE MT1ACTR1 was introduced was solubilized by 50 mM phosphate buffer (pH 7.5) containing 8 M urea, 0.5 M NaC and 10 mM imidazole and was subjected to an Ni⁺⁺ chelating Sepharose column. After washing, it was eluted with 50 mM phosphate buffer (pH 7.5) containing 8 M urea, 0.5 M NaCl and 0.5 M imidazole and the resulting pure fraction was subjected to a PD-10 gel column equilibrated with 20 mM Tris-HCl (pH 8.6) containing 8 M urea and 0.3 M NaCl whereupon the buffer was exchanged. To this were added 50 µM ZnSO₄ and 1 mM DTT, A₂₈₀ thereof was measured and the mixture was diluted with 20 mM Tris-HCl (pH 8.6) containing 8 M urea, 0.3 M NaCl, 50 µM ZnSO₄ and 1 mM DTT so as to make A₂₈₀ 0.1. To this was added 20 mM cysteamine to prepare a sample for re-folding (50 mL). This was dialyzed against 5 L of 50 mM Tris-HCl (pH 8.6) containing 0.15 M NaCl, 5 mM CaCl₂, 100 µM ZnS0₄, 5 mM 2-mercaptoethanol, 1 mM hydroxyethyl disulfide and 0.02% NaN₃ for 16 hours and then dialyzed against 50 mM Tris-HCl (pH 8.6) containing 0.15 M Nacl, 5 mM Cacl₂, 50 µM ZnSO₄ and 0.02% NaN₃ to refold. The sample after the dialysis was centrifuged, insolubilized protein was removed and the residue was concentrated to about 1 mL by ultrafiltration. This was subjected to a Sephacryl S-300 gel column which was equilibrated with 50 mM Tris-HCl buffer (pH 7.5) containing 0.15 M NaCl and 10 mM CaCl₂. The eluted fractions were monitored by means of A₂₈₀ and the fraction into which protein was eluted was detected. The eluted protein showed two peaks and the first half thereof was polymers of dimer or higher ones while the second half thereof was monomers. The protein peak of the latter half was pooled as a pure fraction of MT1-MMP of an activated type and concentrated to 1 mL by ultrafiltration. Protein concentration by BCA (Pierce) was 0.11 mg/mL.

Enzymatic activity of the resulting MT1-MMP of an activated type was investigated.

To 50 µL of the enzyme fraction were added 100 µL of Mca-Pro-Leu-Gly-Leu-Dpa-Ala-Arg-NHs (synthetic substrate for metalloproteinase) prepared into 1 µM by 50 mM Tris-HCl buffer (pH 7.5) containing 0.15 M NaCl, 10 mM CaClₛ, 0.05% Brij 35 and 0.02% NaN₃ and the mixture was allowed to stand at 37°C for 1 hour. At the same time, a product where 20 mM EDTA were added to the above reaction system was prepared and also allowed to stand at 37°C for 1 hour. The reaction solution was placed in a test tube to which 500 µL of 0.1 M sodium acetate buffer (pH 4.0) were added so that the reaction was stopped. Measurement was carried out by a spectrophotofluorometer RF-5000 (Shimadzu) using activity of MT1-MMP for degradating the fluorescent substrate as fluorescent intensity (EI, Ec: 328 nm, Em: 393 nm). The resulting fluorescent intensities were 121.6 and 6.3, respectively and it was confirmed that the prepared MT1-MMP prepared had a metalloproteinase activity inhibited by EDTA. The above-mentioned enzyme concentration, type and concentration of fluorescent substrate, compositions of various reaction solutions, reaction time, reaction temperature and volume of reaction solution can be appropriately changed if necessary.

### ④Assay for MT1-MMP with solid-phase monoclonal anti-MT1-MMP Ab

Antibody solid-phased plates were prepared according to the method mentioned in Example 4 ② . Concentration of each antibody was changed to 50 µg/mL and a blocking solution was changed to 30 mM phosphate buffer (pH 7.0) containing 1% skim milk and 0.1 M NaCl. An enzyme fraction which was diluted to 5 µL/l00 µL with 50 mM Tris-HCl buffer (pH 7.5) containing 0.15 M NaCl, 10 mM CaCl₂, 0.05% Brij 35 and 0.02% NaN₃ was added to an antibody solidifying plate where a blocking solution was previously removed therefrom and washing was conducted for three times using a washing solution (50 mM Tris-HCl buffer (pH 7.4) containing 0.15 M NaCl) and then it was allowed to stand at 4°C for 16 hours. The reaction solution was removed and washed with a washing solution for three times and 100 µL Mca-Pro-Leu-Gly-Leu-Dpa-Ala-Arg-NH₂ (synthetic substrate for metalloproteinase) made into 1 µM with 50 mM Tris-HCl buffer (pH 7.5) containing 0.15 M NaCl 10 mM CaCl₂, 005% Brij 35 and 0.02% NaN₃ were added thereto followed by being allowed to stand at 37°C for 8 hours. The reaction solution was taken into a test tube to which 500 µL of 0.1 M sodium acetate buffer (pH 4.0) were added so that the reaction was stopped. Fluorescent substrate degradation activity trapped with solid-phase monoclonal anti-MT1-MMP antibody and derived from MT1-MMP remaining in the microwell was measured in terms of fluorescence intensity (FI, Ex: 328 nm, Em: 393 nm) by a spectrofluorophotometer RF-5000 (Shimadzu).

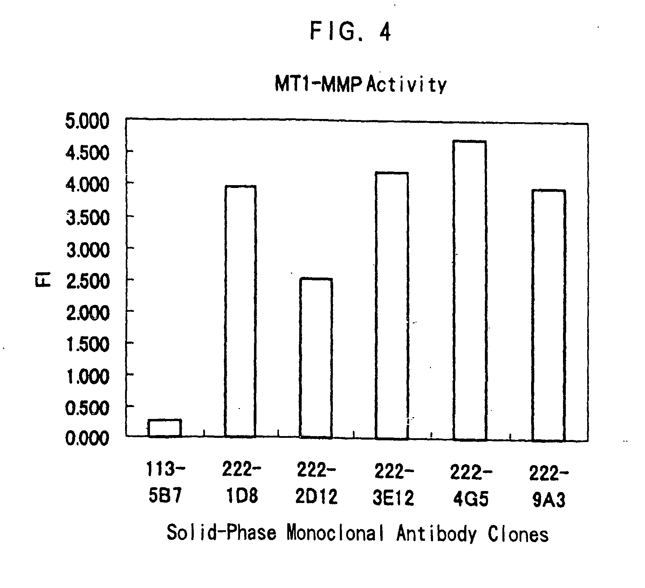

When fluorescent substrate degradation activity was measured using as a solid-phase antibody the antibodies 222-1D8, 222-2D12, 222-3E12, 222-4G5 and 222-9A3 as disclosed herein, there was obtained a fluorescent intensity of 9.2-fold to 17.1-fold than in the case of use of commercially available monoclonal anti-MT1-MMP hemopexin antibody (113-5B7; Daiichi Fine Chemical) (Fig. 4). No enzymatic activity was detected from the product which was similarly prepared without addition of antibody. It was therefore confirmed that, as a result of use of microwell where anti-MT1-MMP antibody is made into solid phase, MT1-MMP was selectively trapped and its activity level was able to be quantitatively detected. Type and concentration of the used antibody, type and concentration of fluorescent substrate, compositions of various reaction solutions, reaction time, reaction temperature, volume of reaction solution, etc. may be appropriately changed upon necessity.

With regard to the antibody which is made into solid phase, appropriate one is an antibody where recognizing region is in hemopexin domain such as 222-1D8, 222-2D12, 222-3E12, 222-4G5 and 222-9A3 although antibodies other than them may be also applicable while there are some inappropriate ones such as 113-5B7. Antibodies where the recognition region is in a catalytic domain may have a possibility of inhibiting the activity of MT1-MMP and they are sometimes inappropriate for the present use. Antibodies where the recognition region is in a propeptide domain may have a possibility of inhibiting the activity of MT1-MMP of an activated type lacking propeptide and they are sometimes inappropriate for the present use.

Any of MT1-MMP of an inactivated type having propeptide domain and MT1-MMP of an activated type lacking propeptide is trapped by microwell where antibody in which recognition region is in hemopexin domain is made into solid phase. When activity is measured under such a state, the MT1-MMP of an activated type in the sample can be selectively measured and, when measurement is carried out after subjecting to an activating treatment with furin or other appropriate protease, surfactant, etc. and then the MT1-MMP of an activated type existing before the activating treatment is deducted, it is now possible to selectively measure the MT1-MMP of an inactivated type in the sample.

### Example 10

### Measurement of Clinical Samples

### (1) Measurement of synovium extract fraction

Synovium tissues were homogenized and centrifuged in 50 mM Tris-HCl buffer (pH 7.5) containing 0.15 M NaCl, 10 mM CaCl₂ and 0.05% Brij 35 to prepare a tissue extract fraction. Concentration of MT1-MMP in the resulting sample was measured according to the method mentioned in Example 4. As a result of measurement of 63 samples, the MT1-MMP concentrations were distributed from not more than 1.3 ng/mL (practical detection limit) to 118.9 ng/mL and the mean value was 23.9 ng/mL.

### (2) Measurement of extract fraction of cancer tissues and normal tissues adjacent to the cancer tissues

Cancer tissues and normal tissues adjacent to the cancer tissues were homogenized and centrifuged in 50 mM Tris-HCl buffer (pH 7.5) containing 0.15 M NaCl, 10 mM CaCl₂ and 0.05% Brij 35 to prepare tissue extract fractions. MT1-MMP concentrations in those samples were measured according to the method mentioned in Example 4. Mean value of 44 cancer tissue extract fractions was 17.2 ng/mL while that of 17 normal tissue extract fractions was not more than 1.3 ng/mL (practical detection limit) whereby it was verified by the sandwich EIA of the present invention that existence of MT1-MMP was much produced in cancer tissues.

### Example 11

### Measurement of Clinical Samples (Lung Cancer Tissues)

### ① Measurement of extract fraction of cancer tissues and normal tissues adjacent to the cancer tissues

About 50 mg each of lung cancer tissues and normal tissues adjacent to the cancer tissues were homogenized and centrifuged in 50 mM Tris-HCl buffer (pH 7.5) containing 0.15 M NaCl, 10 mM CaCl₂ and 0.05% Brij 35 to prepare tissue extract fractions. Concentrations of MT1-MMP in those samples were measured according to the method mentioned in Example 4. Then protein concentration in each tissue extract fraction was measured by BCA (Pierce) and the MT1-MMP concentration was divided by protein concentration to calculate the MT1-MMP amount per protein mass of the sample.

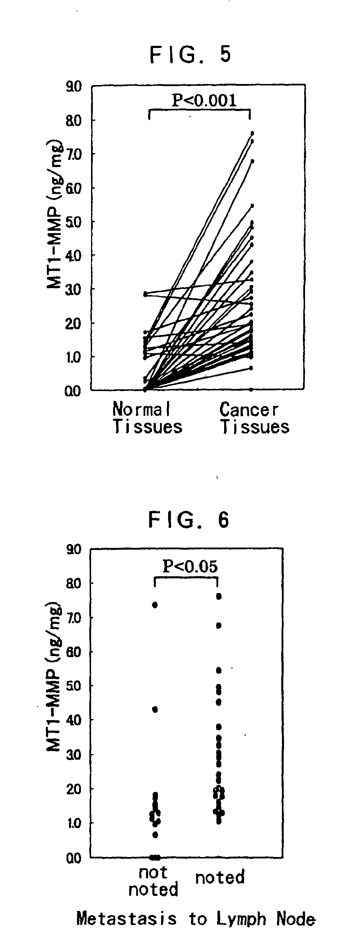

Mean value and standard deviation were determined for 51 samples. The MT1-MMP amounts in the normal tissues and the cancer tissues were 0.41 ± 0.76 ng/mg and 2.43 ± 1.85 ng/mg, respectively and a significant difference (p < 0.001) was confirmed to be between the cancer tissues and the normal tissues (Student's test of significance; Fig. 5).

Similarly were measured the MT1-MMP amount of normal tissues and cancer tissues for head and neck cancer and cancer of the colon as well. MT1-MMP in an amount of about 3.6-fold and 2.4-fold as compared with the adjacent normal tissues was detected from head and neck cancer tissues and colon cancer tissues, respectively. In any of cancer species, significant difference was noted in the MT1-MMP amount between normal tissues and cancer tissues.

### ② Correlation to metastasis

The cancer tissues measured in the above ① were classified using the presence or absence of lymph node metastasis as an index and the correlation between metastasis and MT1-MMP amount was investigated. The MT1-MMP amount (mean value ± standard deviation) of the cancer tissues where no metastasis to lymph node was noted was 1.63 ± 1.777 ng/mg and that where metastasis to lymph node was noted was 2.49 ± 1.78 ng/mg whereupon a significant difference (p < 0.05) was confirmed between both groups (Student's test of significance; Fig. 6).

In Fig. 6, there was a specific sample showing a high MT1-MMP value in spite of the fact that no metastasis to lymph node was noted (the case where no metastasis to lymph node was noted showing the highest value of 7.4 ng/mg). This case was that, although no metastasis to lymph node was noted, strong invasion was noted in vein and malignancy was high. Most of samples showing high MT1-MMP value including that sample showed invasion to lymph node or vessel and, therefore, it can be estimated that, when MT1-MMP level shows a high value even if no metastasis is noted clinically, micrometastasis takes place or there is a high risk of causing metastasis in future.

### Example 12

### Assay for Animal Cell Line-Derived MT1-MMP

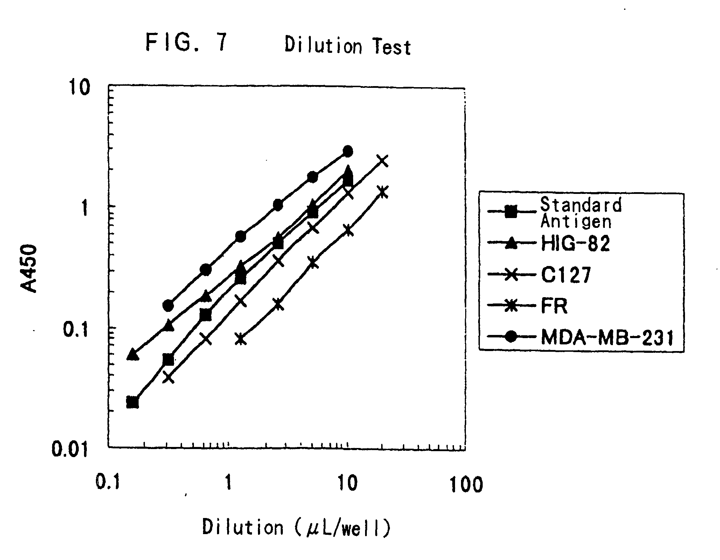

Mouse, rat and rabbit MT1-MMP producing culture cell lines were tested. Culture Supernatants were prepared for each of 8 mouse cell lines, 4 rat cell lines and 1 rabbit cell line in the same manner as in Example 7. The resulting culture supernatant was concentrated to an extent of 20-fold for subjecting to an EIA. Among the animal cell lines, high MT1-MMP production was noted in mouse C127, rat FR and rabbit HIG-82. The concentrated culture supernatant was subjected to a gradient dilution and comparisons were conducted for MT1-MMP-producing human cell line MDA-MB-231 in terms of reactivity. In any of the species, dilution curves (Table 11 and Fig. 7) having the similar inclination were obtained and, therefore, it is noted that each MT1-MMP derived from human, mouse, rat and rabbit cell lines, shows similar immunoreactivity.

**Table 11**

| | A450 | | | | |
|---|---|---|---|---|---|
| Sample Amount ( µL/well) | Standard Antigen | MDA-MB-321 (Human) | C127 (Mouse) | FR (Rat) | HIG-82 (Rabbit) |
| 0.15625 | 0.024 | | | | 0.060 |
| 0.3125 | 0.055 | 0.150 | 0.039 | | 0.105 |
| 0.625 | 0.131 | 0.301 | 0.082 | | 0.185 |
| 1.25 | 0.255 | 0.579 | 0.171 | 0.082 | 0.324 |
| 2.5 | 0.500 | 1.032 | 0.357 | 0.160 | 0.565 |
| 5 | 0.915 | 1.763 | 0.677 | 0.344 | 1.062 |
| 10 | 1.684 | 2.892 | 1.340 | 0.656 | 2.009 |
| 20 | | | 2.520 | 1.348 | |

It was confirmed from the above that the present EIA system is able to measure MT1-MMP derived from not only human being but also various animals such as mouse, rat and rabbit. Amino acid sequences of hemopexin domain of MT1-MMP recognized by the present EIA system were compared in human being (SEQ ID NO: 4), mouse (SEQ ID NO: 5), rat (SEQ ID NO: 6) and rabbit (SEQ ID NO: 7) (Fig. 97). Among the 189 amino acid residues, mismatch was noted only in 1 residue between human being and mouse, 1 residue between human being and rat and 3 residues between human being and rabbit whereby it was confirmed that hemopexin domain was well conserved between species.

### Example 13

### Variation in Immunoreactivity of Antibody in the Presence of a Surfactant and a Reducing Agent

Antibodies were assessed for their immunoreactivity in the presence of surfactants and reducing agents by Western blotting. The standard antigen as used in MT1-MMP sandwich EIA mentioned herein was subjected to SDS-PAGE at 500 ng/lane and transferred to a nitrocellulose membrane. This was blocked with 30 mM phosphate buffer (pH 7.0) containing 1% BSA and 0.1 M NaCl, then dipped in each monoclonal anti-MT1-MMP antibody solution (222-1D8, 222-2D12, 222-3E12, 222-4G5 and 222-9A3) wherein the concentration of each antibody was adjusted to 10 µg/mL with a dilution buffer for MT1-MMP sandwich EIA as disclosed herein and a dilution buffer containing neither SDS nor 2-mercaptoethanol, and incubated for 16 hours. After washing, this was made to react with anti-mouse IgG-HRP for staining.

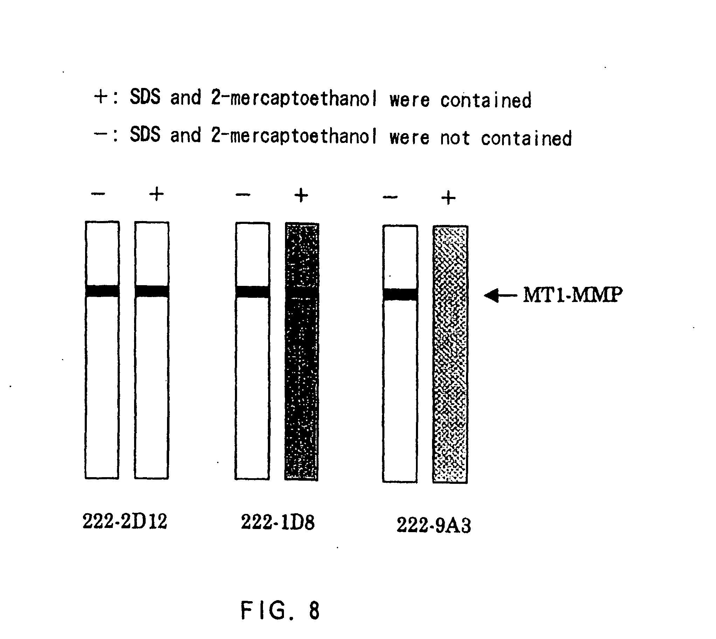

The anti-MT1-MMP monoclonal antibody 222-2D12 used as a solid-phase antibody for MT1-MMP sandwich EIA mentioned herein maintained the reactivity with MT1-MMP regardless of the presence of 2-mercaptoethanol (Fig. 8).

On the other hand, 222-9A3 showed reactivity with SDS similar to 222-2D12 when SDS is contained while, SDS is not contained, reactivity was almost lost (Fig. 8). In the case of 222-1D8 and other antibodies, there was a tendency of losing the specificity such as that reactivity with MT1-MMP significantly lowered or whole membrane was colored. In order to proceed the immunological reaction in a dilution buffer containing surfactants such as SDS and reducing agents such as 2-mercaptoethanol, it is necessary to select an antibody having a stable reactivity such as 222-2D12 used as a solid-phase antibody.

H chain and L chain of human IgG have 4 and 2 S-S bonds, respectively, in the chains and they are bonded each other by means of one S-S bond. In IgG1, the dimers bonded at 2 S-S bonds between H chains so that fourfold structure is stabilized. In IgG3, there are 15 S-S bonds between H chains. When a reducing agent is sufficiently made to act with antibody, S-S bond in and between chain(s) is cleaved and immunoreactivity is lost together with the higher order structure of antibody. It is presumed that IgG3 having more S-S bond as compared with IgG1 has more denaturation effect and that reduction of immunoreactivity is significant. The 222-9A3 which lost its reactivity with MT1-MMP in a dilution buffer for the MT1-MMP sandwich EIA is IgG3. Even in IgG1, cleavage of S-S bond by a reducing agent results in a significant reduction of immunoreactivity but, since H chain and L chain maintain a higher order structure even by interaction due to non-covalent bond, there may be present clone which maintains immunorectivity in the presence of a reducing agent.

### Example 14

### Preparation of Soluble MT1-MMP and Utilization Thereof

There was prepared a PCR primer for amplification of catalytic domain, hinge domain and hemopexin domain (Y¹²⁸C⁵⁵⁵) of MT2-MMP (SwissProt Accession No. 054732; mouse). SmaI site was added to the primer at 5'-side while termination codon and BgIII site were added to the primer at 3'-side. The SmaI site was designed so as to be able to connect in-frame to β-galactosidase of pUC18. pSGMT2-MMP where gene containing full length of MT2-MMP was cloned was used as a template and a PCR was carried out in 30 cycles, of 93°C for 30 seconds, 45°C for 30 seconds and 72°C for 60 seconds. Both ends of the resulting PCR product were cleaved by SmaI and BgIII and inserted into pUC18 which was previously cleaved by SmaI and BamHI. By this expression vector, there was expressed a fusion protein, with a molecular weight of about 48 kDa, comprising total 439 amino acid residues consisting of 11 amino acid residues of β-galactosidase and 428 amino acid residues of Δ MT2-MMP (Y¹²⁸-C⁵⁵⁵) lacking a signal peptide, propeptide region, transmembrane region and intracellular domain.

There was prepared a PCR primer for amplification of catalytic domain, hinge domain and hemopexin domain (y¹²⁰-C⁵³²) of MT3-MMP (SwissProt Accession No. P51512). SmaI site was added to the primer at 5'-side while termination codon and BgIII site were added to the primer at 3'-side. The SmaI site was designed so as to be able to connect in-frame to β-galactosidase of pUC18. pSGMT3-MMP where gene containing full length of MT3-MMP was cloned was used as a template and a PCR was carried out in 30 cycles of 93°C for 30 seconds, 45°C for 30 seconds and 72°C for 60 seconds. Both ends of the resulting PCR product were cleaved by SmaI and BgIII and inserted into pUC18 which was previously cleaved by SmaI and BamHI. By this expression vector, there was expressed a fusion protein, with a molecular weight of about 47 kDa, comprising total 439 amino acid residues consisting of 11 amino acid residues of β-galactosidase and 413 amino acid residues of Δ MT3-MMP (Y¹²⁰-C⁵³²) lacking a signal peptide, propeptide region, transmembrane region and intracellular domain.

There is prepared a PCR primer for amplification of catalytic domain, hinge domain and hemopexin domain (Y¹²⁹-C⁵²⁵) of MT4-MMP (SwissProt Accession No. Q9ULZ9). SmaI site is added to the primer at 5'-side while termination codon and BgIII site are added to the primer at 3'-side. The SmaI site is designed so as to be able to connect in-frame to β-galactosidase of pUC18. Vector or breast cancer cDNA library or the like where gene containing full length of MT4-MMP is cloned is used as a template and a PCR is carried out in 30 cycles of 93°C for 30 seconds, 45°C for 30 seconds and 72°C for 60 seconds. Both ends of the resulting PCR product are cleaved by SmaI and BgIII and inserted into pUC18 which is previously cleaved by SmaI and BamHI. By this expression vector, there is expressed a fusion protein with a molecular weight of about 45 kDa, comprising total 408 amino acid residues consisting of 11 amino acid residues of β-galactosidase and 397 amino acid residues of Δ MT4-MMP (Y¹²⁹Cs2s) lacking a signal peptide, propeptide region, transmembrane region and intracellular domain.

There is prepared a PCR primer for amplification of catalytic domain, hinge domain and hemopexin domain (Y¹⁵⁶-C⁵⁶⁹) of MT5-MMP (SwissProt Accession No. Q9Y5R2). SmaI site is added to the primer at 5'-side while termination codon and BgIII site are added to the primer at 3'-side. The SmaI. site is designed so as to be able to connect in-frame to β -galactosidase of pUC18. Brain cDNA library is used as a template and a PCR is carried out in 30 cycles of 93°C for 30 seconds, 45°C for 30 seconds and 72°C for 60 seconds. Both ends of the resulting PCR product are cleaved by SmaI and BgIII and inserted into pUC18 which is previously cleaved by SmaI and BamHI. By this expression vector, there is expressed a fusion protein, with a molecular weight of about 47 kDa, comprising total 425 amino acid residues consisting of 11 amino acid residues of β-galactosidase and 414 amino acid residues of Δ MT5-MMP (Y¹⁵⁶-C⁵⁶⁹) lacking a signal peptide, propeptide region, transmembrane region and intracellular domain.

There is prepared a PCR primer for amplification of catalytic domain, hinge domain and hemopexin domain (Y¹⁰⁸-C⁵⁰⁸) of MT6-MMP (SwissProt Accession No. Q9NPA2). SmaI site is added to the primer at 5'-side while termination codon and BgIII site are added to the primer at 3'-side. The SmaI site is designed so as to be able to connect in-frame to β -galactosidase of pUC18. Vector where gene containing full length of MT6-MMP is cloned or leukocyte, lung or spleen cDNA library, etc. is used as a template and a PCR is carried out in 30 cycles of 93°C for 30 seconds, 45°C for 30 seconds and 72°C for 60 seconds. Both ends of the resulting PCR product are cleaved by SmaI and BgIII and inserted into pUC18 which is previously cleaved by SmaI and BamHI. By this expression vector, there is expressed a fusion protein, with a molecular weight of about 45 kDa, comprising total 412 amino acid residues consisting of 11 amino acid residues of β-galactosidase and 401 amino acid residues of Δ MT6-MMP (Y¹⁰⁸-C⁵⁰⁸_{'}) lacking a signal peptide, propeptide region, transmembrane region and intracellular domain.

Δ MT2-MMP, Δ MT3-MMP, Δ MT4-MMP, Δ MT5-MMP and Δ MT6-MMP which were recombinant proteins comprising catalytic domain, hinge domain and hemopexin domain were able to be purified and collected by recovering as an inclusion body, solubilizing with 10 mM phosphate buffer (pH 7.0) containing 8 M urea and 0.1 M NaCl and subjecting to a preparative SDS-PAGE and electric elution in accordance with the same method as mentioned in Example 1. Alternatively, a mild re-folding was carried out to prepare MT-MMP having an MMP activity.

When the resulting recombinant protein is used as an immunogen, it is possible to prepare polyclonal antibody and monoclonal antibody. For example, combination of the resulting monoclonal antibody with each recombinant protein (standard antigen) gives sandwich EIA which is able to specifically measure MT2-MMP, MT3-MMP, MT4-MMP, MT5-MMP and MT6-MMP. With regard to solid phase of EIA for the measurement of membrane-associated MT2-MMP, MT3-MMP, MT4-MMP, MT5-MMP and MT6-MMP, it is preferred to select antibody having a resistance to surfactant and reducing agent. Incidentally, it is also possible that membrane-associated MT2-MMP, MT3-MMP, MT4-MMP, MT5-MMP and MT6-MMP which are to be measured are extracted in a buffer containing surfactant and reducing agent.

It is further possible that MT2-MMP, MT3-MMP, MT4-MMP, MT5-MMP and MT6-MMP are made into solid phase via the resulting monoclonal antibody whereupon each MMP activity is measured.

Sandwich EIA of MT2-MMP, MT3-MMP, MT4-MMP, MT5-MMP and MT6-MMP and activity measurement system thereof are able to be applied in various clinical fields such as evaluation of malignancy of cancer, specification of cancer species, detection of organ-specific cancer, estimation of degree of progress of rheumatoid arthritis and Alzheimer's disease, etc. and, moreover, they are able to be utilized as pharmaceuticals such as MT-MMP inhibitor and means for research and development therefor.

### Industrial Applicability

In accordance with the present invention, MT1-MMP can be quickly quantified with high sensitivity and accuracy by the use of simple operation and reagents. Especially in accordance with the present invention, soluble form MT1-MMP in samples to be tested can be quantitated. It is also possible to efficiently screen for compounds or factors which promote or inhibit the expression of MT1-MMP or compounds or factors which promote or inhibit the enzymatic activity of MT1-MMP. It is further possible in the present invention that MT-MMPs are released and/or solubilized from cell membrane and the said MT-MMPs are quantitated. In accordance with the present invention, there are provided reagents, reagent kits and screening kits useful for such a measurement, reagents for cancer or cancer metastasis, agents for testing the progress level of rheumatoid arthritis and Alzheimer's disease, pharmaceuticals such as inhibitors for MT1-MMP activity, means for research and development therefor, etc.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims. This application claims the benefit of Japanese Patent Application No. 2000-352,491 filed on November 20, 2000 that serves as the basis for the right of priority, whose disclosure is hereby incorporated by reference.

## Claims

1. A quantitative immunoassay for a membrane type matrix metalloproteinase (MT-MMP) member selected from the group consisting of membrane type matrix metalloproteinases (MT-MMPs), comprising the use of an antibody to MT-MMP (anti-MT-MMP Ab).

2. The quantitative immunoassay according to claim 1, wherein the assay comprises using an antibody selected from the group consisting of antibodies to MMP-14 (MT1-MMP), antibodies to MMP-15 (MT2-MMP), antibodies to MMP-16 (MT3-MMP), antibodies to MMP-17 (MT4-MMP), antibodies to MMP-24 (MT5-MMP) and antibodies to MMP-25 (MT6-MMP).

3. The quantitative immunoassay according to claim 1 or 2, wherein MT1-MMP is quantitatively immunoassayed with an antibody to MT1-MMP (anti-MT1-MMP Ab).

4. The quantitative immunoassay according to any of claims 1 to 3, wherein the assay comprises using one or more members selected from the group consisting of surfactants and reducing agents.

5. The quantitative immunoassay according to any of claims 1 to 4, wherein the antibody has at least one characteristic property selected from the group consisting of:
(i) abilities of maintaining its immunoreactivity even in the presence of one or more members selected from the group consisting of surfactants and reducing agents and
(ii) products from immunization with a denatured MT-MMP antigen having an epitope artificially exposed.

6. The quantitative immunoassay according to any of claims 1 to 5, wherein the antibody is monoclonal.

7. The quantitative immunoassay according to any of claims 1 to 6, wherein the quantitatively assayable MT-MMP is at least one molecular species selected from the group consisting of:
(i) molecules released and/or solubilized from cell membrane by treatments with one or more members selected from the group consisting of surfactants and reducing agents and
(ii) molecules spontaneously released and/or solubilized from cell membrane.

8. The quantitative immunoassay according to any of claims 1 to 7, wherein the assay comprises the following steps of:
(a) releasing and/or solubilizing MT-MMP from cell membrane by treatment with one or more members selected from the group consisting of surfactants and reducing agents and
(b) quantitatively immunoassaying for MT-MMP.

9. The quantitative immunoassay according to any of claims 4 to 8, wherein the surfactant is sodium dodecylsulfate (SDS).

10. The quantitative immunoassay according to any of claims 4 to 8, wherein the reducing agent is 2-mercaptoethanol.

11. An immunoassay reagent for MT-MMP which is used for the quantitative immunoassay as set forth in any of claims 1 to 10.

12. The reagent according to claim 11, wherein the MT-MMP is a member selected from the group consisting of MT1-MMP, MT2-MMP, MT3-MMP, MT4-MMP, MT5-MMP and MT6-MMP.

13. The reagent according to claim 11, wherein the MT-MMP is MT1-MMP.

14. The reagent according to any of claims 11 to 13, containing an effective amount of one or more members selected from the group consisting of surfactants and reducing agents.

15. A method for releasing and/or solubilizing MT-MMP, which comprises treating a cell membrane-containing sample with one or more members selected from the group consisting of surfactants and reducing agents to release and/or solubilize, from the cell membrane, a member selected from MT-MMPs.

16. The method according to claim 15, wherein the surfactant is SDS.

17. The method according to claim 15, wherein the reducing agent is 2-mercaptoethanol.

18. The method according to any of claims 15 to 17, wherein the MT-MMP is a member selected from the group consisting of MT1-MMP, MT2-MMP, MT3-MMP, MT4-MMP, MT5-MMP and MT6-MMP.

19. The method according to any of claims 15 to 17, wherein the MT-MMP is MT1-MMP.

20. A releasing and/or solubilizing agent for MT-MMP, which is used for (a) the quantitative immunoassay as set forth in any of claims 1 to 10 or (b) the method as set forth in any of claims 15 to 19.

21. The agent according to claim 20, containing an effective amount of one or more members selected from the group consisting of surfactants and reducing agents.

22. The agent according to claim 20 or 21, wherein the MT-MMP is a member selected from the group consisting of MT1-MMP, MT2-MMP, MT3-MMP, MT4-MMP, MT5-MMP and MT6-MMP.

23. The agent according to claim 20 or 21, wherein the MT-MMP is MT1-MMP.

24. A screening method which comprises using one or more elements selected from the group consisting of:
(a) the quantitative immunoassay as set forth in any of claims 1 to 10,
(b) the reagent as set forth in any of claims 11 to 14,
(c) the method as set forth in any of claims 15 to 19, and
(d) the agent as set forth in any of claims 20 to 23, and screening for a compound that promotes or inhibits the expression of a member selected from the group consisting of MT-MMPs.

25. The screening method according to claims 24, wherein the method comprises the following steps:
(1) screening in the presence of a candidate compound to be tested,
(2) screening in the absence of said candidate compound, and
(3) comparing the screening results from the step (1) with those from the step (2).

26. The screening method according to claim 24 or 25, which comprises screening for a compound that promotes or inhibits the expression of MT-MMP selected from the group consisting of MT1-MMP, MT2-MMP, MT3-MMP, MT4-MMP, MT5-MMP and MT6-MMP.

27. The screening method according to claim 24 or 25, which comprises screening for a compound which promotes or inhibits the expression of MT1-MMP.

28. A screening kit for a compound which promotes or inhibits the expression of a member selected from the group consisting of MT-MMPs, said screening kit being used for the screening method as set forth in any of claims 24 to 27.

29. The screening kit according to claim 28, wherein the kit contains an effective amount of one or more elements selected from the group consisting of
(a) an antibody to MT-MMP, said MT-MMP being selected from the group consisting of MT-MMPs,
(b) one or more members selected from the group consisting of surfactants and reducing agents, and
(c) a member selected from the group consisting of MT-MMP attached to a solid phase by an immunological method.

30. The kit according to claim 28 or 29, for the screening of a compound which promotes or inhibits the expression of MT-MMP selected from the group consisting of MT1-MMP, MT2-MMP, MT3-MMP, MT4-MMP, MT5-MMP and MT6-MMP.

31. The kit according to claim 28 or 29, for the screening of a compound which promotes or inhibits the expression of MT1-MMP.

32. A measuring method which comprises assaying for the enzymatic activity of MT-MMP attached to a solid phase by an immunological method.

33. The method according to claim 32, wherein the enzymatic activity of MT-MMP is quantitatively measured.

34. The method according to claim 32 or 33, wherein the solid phase MT-MMP is formed by selectively binding to a solid phase via anti-MT-MMP Ab.

35. The method according to any of claims 32 to 34, wherein the MT-MMP is a member selected from the group consisting of MT1-MMP, MT2-MMP, MT3-MMP, MT4-MMP, MT5-MMP and MT6-MMP.

36. The method according to any of claims 32 to 34, wherein the MT-MMP is MT1-MMP.

37. The method according to any of claims 32 to 36, wherein the MT-MMP is at least one molecular species selected from the group consisting of
(i) molecules released and/or solubilized from cell membrane by treatments with one or more members selected from the group consisting of surfactants and reducing agents and
(ii) molecules spontaneously released and/or solubilized from cell membrane.

38. The method according to any of claims 32 to 37, wherein the method comprises the following steps:
(a) releasing and/or solubilizing MT-MMP from cell membrane by treatments with one or more members selected from the group consisting of surfactants and reducing agents, and
(b) assaying for the enzymatic activity of MT-MMP.

39. Solid phase MT-MMP wherein MT-MMP is attached to a solid phase by an immunological method.

40. The solid phase MT-MMP according to claim 39, wherein MT-MMP is selectively bound to a solid phase via anti-MT-MMP Ab.

41. The solid phase MT-MMP according to claim 40, wherein the anti-MT-MMP Ab is an antibody which recognizes a hemopexin-like domain of MT-MMP.

42. The solid phase MT-MMP according to any of claims 39 to 41, wherein MT-MMP is a member selected from the group consisting of MT1-MMP, MT2-MMP, MT3-MMP, MT3-MMP, MT4-MMP, MT5-MMP and MT6-MMP.

43. The solid phase MT-MMP according to any of claims 39 to 41, wherein MT-MMP is MT1-MMP.

44. A screening method for a compound which promotes or inhibits the enzymatic activity of MT-MMP which comprises using (i) the method as set forth in any of claims 32 to 38 or (ii) the solid phase MT-MMP as set forth in any of claims 39 to 43.

45. The screening method according to claim 44, wherein the method comprises the following steps:
(1) screening in the presence of a candidate compound to be tested,
(2) screening in the absence of said candidate compound, and
(3) comparing the screening results from the step (1) with those from the step (2).

46. A screening kit for a compound which promotes or inhibits the enzymatic activity of MT-MMP, said kit being used for the method as set forth in any of claims 32 to 38

47. The kit according to claim 46, comprising an effective amount of solid phase MT-MMP as set forth in any of claims 39 to 43.

48. A pharmaceutical drug or composition comprising an effective amount of one or more elements selected from the group consisting of
(a) a compound which is obtained or identified by the method as set forth in any of claims 24 to 27, said compound which promotes or inhibits the expression of a member selected from the group consisting of MT-MMPs,
(b) a compound which is obtained or identified by the use of the kit as set forth in any of claims 28 to 31, said compound which promotes or inhibits the expression of a member selected from the group consisting of MT-MMPs,
(c) a compound which is obtained or identified by the method as set forth in claim 44 or 45, said compound which promotes or inhibits the enzymatic activity of MT-MMP, and
(d) a compound which is obtained or identified by the use of the kit as set forth in claim 46 or 47, said compound which promotes or inhibits the enzymatic activity of MT-MMP.

49. The pharmaceutical drug or composition according to claim 48, comprising an effective amount of (i) a compound that promotes or inhibits the expression of MT-MMP, said MT-MMP being selected from the group consisting of MT1-MMP, MT2-MMP, MT3-MMP, MT4-MMP, MT5-MMP and MT6-MMP, or (ii) a compound that promotes or inhibits the enzymatic activity of said MT-MMP.

50. The pharmaceutical drug or composition according to claim 48, comprising an effective amount of (i) a compound that promotes or inhibits the expression of MT1-MMP, or (ii) a compound that promotes or inhibits the enzymatic activity of MT1-MMP.

51. A diagnostic or testing drug for cancer or for cancer metastasis, enabling an assay for a member selected from the group consisting of MT-MMPs with (i) the quantitative immunoassay as set forth in any of claims 1 to 10 or (ii) the method as set forth in any of claims 32 to 38.

52. The diagnostic or testing drug according to claim 51, with which
(A) the level of MT-MMP is used as an indicator for evaluating cancer malignancy or for predicting cancer metastatic potential, said MT-MMP being at least one molecular species selected from
(i) molecules released and/or solubilized from cell membrane by treatments with one or more members selected from surfactants and reducing agents, and
(ii) molecules spontaneously released and/or solubilized from cell membrane,
or
(B) the enzymatic activity level of MT-MMP is used as an indicator for evaluating cancer malignancy or for predicting cancer metastatic potential, said MT-MMP being at least one molecular species selected from
(iii) molecules released and/or solubilized from cell membrane by treatments with one or more members selected from surfactants and reducing agents, and
(iv) molecules spontaneously released and/or solubilized from cell membrane.

53. The diagnostic or testing drug according to claim 51 or 52, enabling an assay for MT-MMP selected from the group consisting of MT1-MMP, MT2-MMP, MT3-MMP, MT4-MMP, MT5-MMP and MT6-MMP.

54. The diagnostic or testing drug according to claim 51 or 52, enabling an assay for MT1-MMP.

55. The diagnostic or testing drug according to any of claims 51 to 53, comprising an effective amount of one or more antibodies selected from the group consisting of antibodies to MT1-MMP, antibodies to MT2-MMP, antibodies to MT3-MMP, antibodies to MT4-MMP, antibodies to MT5-MMP and antibodies to MT6-MMP.

56. The diagnostic or testing drug according to any of claims 51, 52 and 54, comprising an effective amount of an antibody to MT1-MMP.

57. The diagnostic or testing drug according to any of claims 51, 52, 54 and 56, enabling an assay for the enzymatic activity of MT1-MMP.

58. An antibody which is reactive to MT-MMP wherein the MT-MMP is selected from the group consisting of MT-MMPs, said antibody having at least one characteristic property selected from the group consisting of
(i) abilities of maintaining its immunoreactivity even in the presence of one or more members selected from the group consisting of surfactants and reducing agents, and
(ii) products from immunization with a denatured MT-MMP antigen having an epitope artificially exposed.

59. The antibody according to claim 58, being selected from the group consisting of antibodies to MT1-MMP, antibodies to MT2-MMP, antibodies to MT3-MMP, antibodies to MT4-MMP, antibodies to MT5-MMP and antibodies to MT6-MMP.

60. The antibody according to claim 58 or 59, wherein the antibody is monoclonal.
